(19)

Europäisches Patentamt

European Patent Office

Office européen des brevets

(11) **EP 2 202 249 A1**

(12) **EUROPEAN PATENT APPLICATION**

(43) Date of publication:
**30.06.2010 Bulletin 2010/26**

(51) Int Cl.:
*C08F 20/06* (2006.01)  *A61L 15/22* (2006.01)
*C08J 3/16* (2006.01)

(21) Application number: **10155064.8**

(22) Date of filing: **01.11.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.03.2006 JP 2006097028**

(83) **Declaration under Rule 32(1) EPC (expert solution)**

(62) Document number(s) of the earlier application(s) in accordance with Art. 76 EPC:
**06822789.1 / 2 006 306**

(71) Applicant: **Asahi Kasei Chemicals Corporation Tokyo 101-8101 (JP)**

(72) Inventors:
- **Higashimoto, Naonori**
  **Tokyo 101-8101 (JP)**
- **Kodama, Tamotsu**
  **Tokyo 101-8101 (JP)**
- **Nishi, Masataka**
  **Tokyo 101-8101 (JP)**

(74) Representative: **Bradley, Josephine Mary**
**D Young & Co LLP**
**120 Holborn**
**London EC1N 2DY (GB)**

Remarks:
This application was filed on 01-03-2010 as a divisional application to the application mentioned under INID code 62.

(54) **Water-absorbing resin particle agglomerates**

(57)   Provided are water absorbing resin particle agglomerates stably showing a high water retention property and satisfying the following requirements:(a) 50 mol% or greater of repeating units of the polymer molecular chain of the water absorbing resin constituting the primary particles are carboxyl group-containing units and at least a portion of carboxyl groups of the carboxyl group-containing units is neutralized with at least one base selected from alkali metals, amines, and ammonia, and (b) the water absorbing resin particle agglomerates comprise, on the outer surface thereof, a portion having a neutralization ratio of carboxyl groups of not greater than 40 mol% and, inside of the water absorbing resin particle agglomerates, a portion having a neutralization ratio of carboxyl groups of 50 mol% or greater.

EP 2 202 249 A1

**Description**

Technical Field

The present invention relates to water absorbing resin particle agglomerates suited for use in absorbents used for various purposes such as hygiene materials including disposable diapers, sanitary napkins, and incontinence pads; and a production process of the water absorbing resin particle agglomerates.

Background Art

As one of synthetic polymers, a water absorbing resin which gels by absorbing a large amount of water has been developed and it is used widely in the fields of hygiene materials such as paper diapers and sanitary napkins, fields of agriculture and forestry, and civil engineering fields. As such a water absorbing resin, many resins are known, for example, crosslinked partially-neutralized polyacrylic acid (refer to, for example, Patent Document 1), hydrolysate of starch-acrylonitrile graft polymer (refer to, for example, Patent Document 2), neutralized product of starch-acrylic acid graft polymer (refer to, for example, Patent Document 3), saponified product of vinyl acetate-acrylate copolymer (refer to, for example, Patent Document 4), and hydrolysate of an acrylonitrile copolymer or acrylamide copolymer (refer to, for example, Patent Document 5).

In recent years, there is an increasing demand for paper diapers for the aged with an increase in the average life and an absorbent is required to have a higher water retention property.
At present, however, resins composed mainly of sodium polyacrylate and used ordinarily as a water absorbing resin which is a material for absorbents have an absorption ratio of approximately 60 g/g for 0.9% physiological saline and this value is an upper limit of the water retention property of these resins. Accordingly, they do not have a sufficient water retention property.

Water absorbing resin particles having improved absorption performance under pressure are known (for example, Patent Document 6). They are obtained by controlling a neutralization ratio of carboxyl groups inside the particles to a specific value or greater and a neutralization ratio of carboxyl groups on the outer surface of the particles to not greater than a specific value.
The water absorbing resin particles disclosed in Patent Document 6 however have a water absorption ratio under no pressure of approximately 60 g/g and do not have a sufficient water retention property.

In addition, water absorbing resins have posed problems due to fine dust generated when an absorbent is produced using them, that is, health problem of workers who are engaged in the production work and may suck fine dust, environmental problems, and adverse effect on the production equipment. Various methods for increasing the particle size of water absorbing resins have been studied conventionally.

Reversed-phase suspension polymerization methods using a special surfactant (for example, Patent Documents 7, 8, 9, and 10) have also been investigated for the production of water absorbing resin particles having a large particle size. The particle size obtainable by these methods however is only several hundred μm or so and these methods have problems such as difficulty in procuring a surfactant suited for use, lack of stability of an emulsion in polymerization, and a low absorption ratio.

It has also been studied to enlarge the particle size by agglomerating primary particles of a water absorbing resin (for example, Patent Documents 11 to 15).

In the methods described in Patent Documents 11 and 12, primary particles formed by polymerization are agglomerated into secondary particles during polymerization in the presence of inorganic powders or under azeotropic dehydration conditions. Mixing of the inorganic powders which are foreign matters is not preferred in the field of hygiene materials.
In the methods described in Patent Documents 13 and 14, on the other hand, primary particles formed by polymerization are agglomerated into secondary particles by azeotrpic dehydration in the presence of a polyalkylene glycol. This method requires azeotropic dehydration and due to a large energy loss caused thereby, it does not achieve a high production efficiency.

The method disclosed in Patent Document 15 increases the particle size by the agglomeration of particles by two-stage polymerization method. It is a method in which polymerization is performed in two stages so that production efficiency is inferior to one-stage polymerization.

As described above, a method which is convenient and at the same time, enables production of water absorbing resin particles having a sufficiently high water retention property and having a large particle size without using a special material is hitherto unknown.

Patent Document 1: Japanese Patent Laid-Open No. Sho 55-84304

Patent Document 2: Japanese Patent Publication No. Sho 49-43395
Patent Document 3: Japanese Patent Laid-Open No. Sho 51-125468
Patent Document 4: Japanese Patent Laid-Open No. Sho 52-14689
Patent Document 5: Japanese Patent Publication No. Sho 53-15959
Patent Document 6: Japanese Patent Laid-Open No. 2005-200630
Patent Document 7: Japanese Patent Publication No. Hei 6-6612
Patent Document 8: Japanese Patent Publication No. Hei 1-17482
Patent Document 9: Japanese Patent Publication No. Sho 63-36322
Patent Document 10: Japanese Patent Publication No. Sho 63-36321
Patent Document 11: Japanese Patent Laid-Open No. Sho 62-132936
Patent Document 12: Japanese Patent Publication No. Hei 3-26204
Patent Document 13: U.S. Patent No. 6586534
Patent Document 14: U.S. Patent No. 6174946
Patent Document 15: Japanese Patent Laid-Open No. Hei 9-77810

Disclosure of the Invention

Problems to be solved by the Invention

An object of the present invention is to provide a convenient production process of water absorbing resin particles which process can produce water absorbing resin particles having a high water retention property and a large particle size without using a special material.
Another object of the present invention is to provide water absorbing resin particle agglomerates that exhibit a high water retention property stably.

Means for Solving the Problems

The present inventors have found that in manufacturing a water absorbing resin by reverse-phase suspension polymerization of an unsaturated carboxylate in the presence of a surfactant, after formation of primary particles of the water absorbing resin, the primary particles agglomerate by the addition of a water soluble solvent and thereby facilitates the formation of water absorbing resin agglomerates; and that the agglomerates obtained by this method have an enhanced water retention property compared with that of the primary particles.
The present inventors have also found that water absorbing resin particle agglomerates obtained by adjusting the neutralization ratio of carboxyl groups on the outer surface and inside of the agglomerates to predetermined values, respectively, exhibit a high water retention property stably without causing gel blocking.
In a first aspect of the present invention, there is thus provided a manufacturing method of water absorbing resin particle agglomerates, which comprises the following steps (1) and (2):

(1) a polymerization step for producing primary particles of a water absorbing resin comprising suspending an aqueous monomer solution containing an unsaturated carboxylate in an organic solvent containing a nonionic surfactant therein, and subjecting the resulting suspension to reverse-phase suspension polymerization; and
(2) an agglomeration step of agglomerating the primary particles by using a water soluble solvent.

In a second aspect of the present invention, there is also provided water absorbing resin particle agglomerates of comprising primary particles consisting of a water absorbing resin and satisfying the following requirements (a) and (b):

(a) 50 mol% or greater of repeating units of the polymer molecular chain of the water absorbing resin constituting the primary particles are carboxyl group-containing units and at least a portion of carboxyl groups of the carboxyl group-containing units is neutralized with at least one base selected from alkali metals, amines, and ammonia, and
(b) the water absorbing resin particle agglomerates comprise, on the outer surface thereof, a portion having a neutralization ratio of carboxyl groups of not greater than 40 mol% and, inside of the water absorbing resin particle agglomerates, a portion having a neutralization ratio of carboxyl groups of 50 mol% or greater.

The reason why the water absorbing resin particle agglomerates having a high water retention property can be prepared according to the first aspect of the present invention has not been elucidated but it is presumed as follows.
It is presumed that by the agglomeration of the primary particles, water is enclosed in spaces formed by the agglomerated primary particles, resulting in improvement of a water retention property.
In addition, it is presumable that the surface area is increased during water absorption by dropout of a portion of the primary particles from secondary particles during water absorption, thereby increasing the absorption rate more than the case of increasing the absorption rate by enlarging the size of the primary particles.

Moreover, it is presumable that release of a neutral salt of an unsaturated carboxylic acid from the surface of resin particles is reduced by agglomerating a number of the primary particles, thereby prevents deterioration in the water retention property.

Described specifically, the water retention property of a water absorbing resin obtained by polymerization of an unsaturated carboxylate depends on the amount of a neutral salt (electrolyte) in the resin, however the neutral salt is apt to be released from the surface of the resin by heating or the like. In the water absorbing resin particle agglomerates produced by the first embodiment of the present invention, however, primary particles agglomerate and their surface area exposed to outside is small so that release of the neutral salt can be reduced.

It is presumable further that the water absorbing resin particle agglomerates of the present invention have an improved water retention property because the water absorbing resin constituting the agglomerates is modified by a water soluble solvent, preferably an alcohol, that is used during the agglomeration of the primary particles.

More specifically, it is presumable that in the agglomeration step, the hydrophilic group and the hydrophobic group of the polymer chain of the water absorbing resin start to move toward the hydrophilic group and the hydrophobic group of the water soluble solvent, respectively, which loosens the entanglement between the polymer chains, reduces the number of crosslink points which restrict the water retention property, and enhances the water retention property.

The reason why the water absorbing resin particle agglomerates according to the second embodiment of the present invention show a stable high water retention property has not been elucidated, but is presumable as follows.

As described above regarding the first embodiment of the present invention, by agglomerating the primary particles, water is enclosed in the space formed between agglomerated primary particles and the agglomerates have an improved water retention property.

In addition, it is presumable that the surface area is increased during water absorption by dropout of a portion of the primary particles from secondary particles during water absorption, thereby increasing the absorption rate more than the case of increasing the absorption rate by enlarging the size of the primary particles.

In particular, when the agglomerated secondly particles have a high proportion of particles having a large particle size such as those used in a water absorbing composite such as body liquid absorbing goods, , the agglomerated secondly particles sometimes adhere to each other during water absorption and cause a gel blocking phenomenon called *"Mamako* phenomenon"*. Occurrence of such gel blocking inhibits penetration of water between the particles and as a result, the agglomerates cannot completely exhibit their water absorbing capacity and as a result, fail to achieve a high water retention property. The reason why this gel blocking occurs is presumed that the outer surfaces of the particles swollen during water absorption are apt to adhere to each other.

As described above, however, while the water retention property of a water absorbing resin is thought to depend on the amount of a neutral salt, in the second embodiment of the present invention, it is presumed that due to a reduction in a neutralization ratio of at least some carboxyl groups on the outer surface of the agglomerates, the water retention property on the outer surface decreases and the particles do not swell so much during water absorption and therefore gel blocking between particles can be suppressed.

In addition, in the second embodiment of the present invention, the agglomerates still have a high water absorbing capacity because they have, inside thereof, a portion with a high neutralization ratio of carboxyl groups.

Accordingly, the agglomerates as a whole can achieve a high water retention property stably without causing gel blocking.

Effect of the Invention

According to the first embodiment of the present invention, a water absorbing resin material having a high water retention property (water absorption ratio) and a high absorption speed can be provided.

In addition, the first embodiment of the present invention facilitates the control of the particle size of the agglomerated secondary particles so that a water absorbing resin material having a large particle size and having no adverse effect on the health and environment can be provided

The water absorbing resin particle agglomerates according to the second embodiment of the present invention can achieve a high water retention property (water absorption ratio) stably even if the agglomerates have a high proportion of large particles.

Best Mode for Carrying out the Invention

The manufacturing method of the first embodiment of the present invention can be roughly separated into a polymerization step, an agglomeration step, a fusion bonding step, a collection step, a drying step, and a heating step. Each of these steps will hereinafter be described more specifically.

(Polymerization step)

In the first embodiment of the present invention, reverse-phase suspension polymerization in which the polymerization is carried out while an aqueous monomer solution containing an unsaturated carboxylate is suspended in an organic solvent is employed. The reactor used for it is not limited particularly and either one of a batch system or a continuous system can be employed. Examples include a loop reactor and an ordinarily employed stirring tank.

The term "unsaturated carboxylate" means a salt obtained by neutralizing an unsaturated carboxylic acid with an alkali metal, ammonia or amines. One or more types of unsaturated carboxylates may be used singly or in a combination of two or more.

Preferred examples of the unsaturated carboxylates include, from the viewpoint of increasing an absorption ratio of water absorbing resin prepared therefrom, ammonium salts, sodium salts, and lithium salts. Of these, ammonium salts and sodium salts are preferred in view of the influence on human bodies and ammonium salts are more preferred in view of both the influence on human bodies and an absorption ratio.

An unsaturated carboxylic acid ammonium salt may partially contain an unsaturated carboxylic acid amide. The term "unsaturated amide" means a compound having, in the molecule thereof, both an unsaturated bond and a functional group represented by the following formula: RCONH- (R representing any organic group such as alkyl or aryl). Examples of such a compound include cinnamic acid amide, acrylamide, and methacrylamide. Of these, acrylamide and methacrylamide are preferred, with acrylamide being more preferred.

The term "unsaturated carboxylic acid" as used herein means a compound having both an unsaturated bond and a carboxylic acid group. It may contain many unsaturated bonds and many carboxylic acid groups. The term "unsaturated bond" means a double bond (ethylene bond) or a triple bond (acetylene bond) between carbon atoms. Examples of the unsaturated carboxylic acid for the preparation of an ammonium salt include (meta)acrylic acid, methacrylic acid, maleic acid, fumaric acid, itaconic acid, crotonic acid, and cinnamic acid. Of these unsaturated carboxylic acids, acrylic acid and methacrylic acid are preferred from the viewpoint of polymerizability and absorption property of the polymer obtained therefrom.

Unsaturated carboxylic acid ammonium salts which are preferred examples of the unsaturated carboxylates in the first embodiment of the present invention may be prepared in any manner. Examples include a. hydrolysis of an unsaturated nitrile and/or an unsaturated amide with a microorganism and b. neutralizing an unsaturated carboxylic acid with ammonia.

a. Hydrolysis with microorganism

The unsaturated nitrile to be hydrolyzed with a microorganism means a compound having, in the molecule thereof, both an unsaturated bond and a cyan group. It may have many unsaturated bonds and many cyan groups. The term "unsaturated bond" means a double bond (ethylene bond) or a triple bond (acetylene bond) between carbon atoms. Examples of such a compound include acrylonitrile, methacrylonitrile, crotonitrile, and cinnamic acid nitrile. Of these, acrylonitrile and methacrylonitrile are preferred, with acrylonitrile being more preferred.

The unsaturated amide to be hydrolyzed with a microorganism means a compound having, in the molecule thereof, both an unsaturated bond and a functional group represented by the formula: RCONH- (R representing any organic group such as alkyl or aryl). Examples of such a compound include cinnamic acid amide, acrylamide, and methacrylamide, of which acrylamide and methacrylamide are preferred, with acrylamide being especially preferred.

Hydrolysis conditions of the unsaturated nitrile and/or the unsaturated amide with a microorganism are not particularly limited, and microorganisms that are capable of producing an aqueous solution of an unsaturated carboxylic acid ammonium salt having a concentration of 20 wt.% or greater are preferred. As such a microorganism, at least one microorganism selected from the group consisting of the genus *Acinetobacter*, the genus *Alcaligenes*, the genus *Corynebacterium*, the genus *Rhodococcus*, and the genus Gordona is preferred. Of these microorganisms, those belonging to the genus *Acinetobacter* are preferred, and the following strain deposited by Asahi Kasei Chemicals (1-1-2, Yuraku-cho, Chiyoda-ku, Tokyo, Japan) is preferred.

(1) *Acinetobacter sp.* AK226 strain of an accession number FERM BP-08590 deposited with The International Patent Organism Depositary, National Institute of Advanced Industrial Science and Technology, Independent Administrative Institution (Central 6, 1-1-1 Higashi, Tsukuba-shi, Ibaraki, Japan (Postal code: 305-8566)) on January 7, 2004 (date of original deposit).

*(2) Acinetobacter sp.* AK227 strain of an accession number FERM BP-08591 deposited with The International Patent Organism Depositary, National Institute of Advanced Industrial Science and Technology, Independent Administrative Institution (Central 6, 1-1-1 Higashi, Tsukuba-shi, Ibaraki, Japan (Postal code: 305-8566)) on January 7, 2004 (date of original deposit).

The microbial properties of *Acinetobacter sp.* AK226 strain (FERM BP-08590) and *Acinetobacter sp.* AK227 strain (FERM BP-08591) are as shown below in Table 1.

[Table 1]

| | AK226 | AK227 |
|---|---|---|
| Morphology 1. Shape and size of cells | Rod-shaped bacteria From 1.0 to 1.2 × from 1.4 to 2.7 μm | Rod-shaped bacteria From 1.0 to 1.6 × from 1.5 to 2.6 μm |
| 2. Polymorphism of cells | None | None |
| 3. Motility | None | None |
| 4. Spore | None | None |
| 5. Gram stain | - | - |
| 6. Acid resistance | - | - |
| Growth state in each culture medium 1. Broth agar plate culture | Circular, translucent, with gloss, pale yellowish white | Circular, translucent, with gloss, pale yellowish white |
| 2. Broth agar slant culture | Medium degree of growth, smooth surface, with gloss, translucent, pale yellowish white | Medium degree of growth, smooth surface, with gloss, translucent, pale yellowish white |
| 3. Broth liquid culture | Pellicle formation, medium degree of growth, with sediment | Pellicle formation, medium degree of growth, with sediment |
| 4. Broth gelatin stab culture | Good growth on the surface, no liquefaction | Good growth on the surface, no liquefaction |
| 5. Litmus milk | No change | No change |
| Physiological properties 1. Reduction of nitrate | - | - |
| 2. Denitrification reaction | - | - |
| 3. MR test | - | - |
| 4. VP test | - | - |
| 5. Indole formation | - | - |
| 6. Hydrogen sulfide formation | - | - |
| 7. Hydrolysis of starch | - | - |
| 8. Utilization of citric acid | Cinnamon medium + | Cinnamon medium + |
| 9. Utilization of inorganic nitrogen source | Sulfate - Ammonium salt - | Sulfate - Ammonium salt - |
| 10. Pigment formation | King-A medium - King-B medium - | King-A medium - King-B medium - |
| 11. Urease | - | - |
| 12. Oxidase | - | - |
| 13. Catalase | + | + |

(continued)

| | AK226 | | AK227 | |
|---|---|---|---|---|
| 14. Hydrolysis of cellulose | - | | - | |
| 15. Range of growth | pH: from 5 to 12 Temperature: from 10 to 40°C | | pH: from 5 to 12 Temperature: from 10 to 45°C | |
| 16. Behavior in oxygen | Aerobic | | Aerobic | |
| 17. O-F test | - | | - | |
| 18. Heat resistance 10% Skimmed milk | Completely killed at 55°C/15 min. | | Almost killed at 55°C/15 min. | |
| 19. Acid and gas formation from sugar | Acid formation | Gas formation | Acid formation | Gas formation |
| L-arabinose | - | - | - | - |
| D-xylose | - | - | - | - |
| D-glucose | - | - | - | - |
| D-mannose | - | - | + | - |
| D-fructose | - | - | - | - |
| Sucrose | - | - | - | - |
| Lactose | - | - | - | - |
| Trehalose | - | - | - | - |
| D-sorbitol | - | - | - | - |
| D-mannitol | - | - | - | - |
| Inositol | - | - | - | - |

The aqueous solution of an unsaturated carboxylic acid ammonium salt prepared by hydrolysis using the above-described microorganism contains a considerably trace amount of impurities such as a dimer and/or hydrate of the unsaturated carboxylic acid so that this hydrolysis is preferred.
Specific examples of the impurities include, when the unsaturated carboxylic acid is acrylic acid, β-acryloyloxypropionic acid which is a dimer of acrylic acid and β-hydroxypropionic acid which is a hydrate of acrylic acid, and salts thereof.

b. Neutralization of an unsaturated carboxylic acid with ammonia

An unsaturated carboxylic acid used in the neutralization of the unsaturated carboxylic acid with ammonia is same as the above-described unsaturated carboxylic acids.
The unsaturated carboxylic acid may be prepared in any preparation method. When a large amount of impurities is contained in such an unsaturated carboxylic acid, it is preferred to reduce its impurity content by purification. The term "impurities" as used herein means compounds which may be decomposed and may become a monomer component. Examples include compounds having a hydrated unsaturated bond and oligomers. The impurities contained in acrylic acid are, for example, β-hydroxypropionic acid and β-acryloyloxypropionic acid. Any purification method can be employed insofar as it can reduce the impurity content to a specified amount or less and a purification means is not limited particularly. For example, distillation may be employed. The impurity content is reduced to preferably 1000 ppm or less, more preferably 500 ppm or less, still more preferably 300 ppm or less, most preferably 100 ppm or less. An excessively large impurity content is not preferred, because a large amount of residual monomers remain in the obtained water absorbing resin, and the residual monomer increase in subsequent steps of manufacturing method, and moreover, various physical properties of the polymer may become unsatisfactory.
A neutralization method is not particularly limited and either aqueous ammonia or an ammonia gas may be used. Neutralization may be performed under conditions so that a neutralization ratio of acrylic acid exceeds 100 mol% at least once during a certain period of the neutralization step. In the neutralization step, the temperature is maintained preferably at from 0 to 50°C by cooling. Excessive increase in the temperature is not preferred because it may inevitably produce β-hydroxypropionic acid or oligomer.
An amount of the alkali metal salt of an unsaturated carboxylic acid in an aqueous monomer solution is preferably from 0 to 45 mol% relative to the total amount in moles of the unsaturated carboxylic acid and salts thereof (sum of the amount in moles of an unsaturated carboxylic acid ammonium salt, the alkali metal salt of an unsaturated carboxylic acid, and an unsaturated carboxylic acid). The mol% of the alkali metal salt of an unsaturated carboxylic acid contained in the aqueous monomer solution is preferably smaller in order to improve an absorption ratio of a water absorbing resin produced and it is more preferably from 0 to 20 mol%, still more preferably form 0 to 10%.

The amount of the unsaturated carboxylic acid ammonium salt in the aqueous monomer solution is preferably from 60 to 100 mol% relative to the total amount in moles of the unsaturated carboxylic acids and salts thereof (the sum of the amount in moles of the unsaturated carboxylic acid ammonium salt, the alkali metal salt of an unsaturated carboxylic acid, and an unsaturated carboxylic acid) from the viewpoint of the absorption ratio of a water absorbing resin thus produced. The mol% of the unsaturated carboxylic acid ammonium salt contained in the aqueous monomer solution is preferably higher in order to improve the absorption ratio of the water absorbing resin thus produced and the mol% is more preferably from 80 to 100 mol%, still more preferably from 95 to 100%.

The aqueous monomer solution may contain an unsaturated carboxylic acid. The amount of the unsaturated carboxylic acid to be added is preferably from 0 to 45 mol% relative to the total amount in moles of the monomers (sum of the moles of the unsaturated carboxylic acid ammonium salt, the alkali metal salt of an unsaturated carboxylic acid, the unsaturated carboxylic acid, and the other monomer(s)). In order to improve the absorption ratio of the water absorbing resin produced, the mol% of the unsaturated carboxylic acid is preferably lower. It is more preferably from 0 to 20 mol%, still more preferably from 0 to 10%.

The aqueous monomer solution may contain monomers other than the unsaturated carboxylic acid and salts thereof. The other monomers are mainly monofunctional unsaturated monomers. Examples include hydrophilic monofunctional unsaturated monomers containing an acid group such as vinylsulfonic acid, styrenesulfonic acid, 2-(meth) acrylamido-2-methylpropanesulfonic acid, 2-(meth)acryloylethanesulfonic acid, and 2-(meth)acryloylpropanesulfonic acid, and salts thereof; hydrophilic monofunctional unsaturated monomers containing an amide group such as acrylamide, methacrylamide, N-ethyl (meth)acrylamide, N-n-propyl (meth)acrylamide, N-isopropyl (meth)acrylamide and N,N-dimethyl (meth)acrylamide; esterified hydrophilic unsaturated monomers such as 2-hydroxyethyl (meth)acrylate, 2-hydroxypropyl (meth)acrylate, methoxypolyethylene glycol (meth)acrylate, and polyethylene glycol mono(meth)acrylate; N-atom-containing hydrophilic monofunctional unsaturated monomers such as vinylpyridine, N-vinylpyrrolidone, N-acryloylpiperidine, N-acryloylpyrrolidine, N,N-dimethylaminoethyl (meth)acrylate, N,N-diethylaminoethyl (meth)acrylate, N,N-dimethylaminopropyl (meth)acrylate, and N,N-dimethylaminoethyl (meth)acrylamide, and quaternary salts thereof; and hydrophobic monofunctional unsaturated monomers such as styrene, vinyl chloride, butadiene, isobutene, ethylene, propylene, and alkyl (meth)acrylate.

Of these, (meth)acrylic acid (salt thereof), 2-(meth)acryloylethanesulfonic acid (salt thereof), 2-(meth)acrylamido-2-methylpropanesulfonic acid (salt thereof), methoxypolyethylene glycol (meth)acrylate, N,N-dimethylaminoethyl (meth)acrylate, and (meth)acrylamide are preferred.

The content of these monomers other than the unsaturated carboxylic acid and salts thereof is preferably from 0 to 45 mol% relative to the total amount in moles of the monomers (sum of the moles of the unsaturated carboxylic acid ammonium salt, the alkali metal salt of an unsaturated carboxylic acid, the unsaturated carboxylic acid, and the other monomer(s)). These monomers are used for modifying the water absorbing resin depending on various purposes so that the optimum using amount differs depending on the using purpose. In order to prevent a reduction in the absorption ratio of the water absorbing resin, however, the using amount is preferably smaller. It is preferably from 0 to 20 mol%, more preferably from 0 to 5 mol%.

In the present invention, a crosslinked structure may be introduced into the water absorbing resin by using a radical polymerizable crosslinking agent at the time of polymerization. The radical polymerizable crosslinking agent is not limited insofar as it is a compound having, in one molecule thereof, a plurality of polymerizable unsaturated groups and/or reactive groups. Use of a compound having a high hydrophilicity as a radical polymerizable crosslinking agent is preferred because it improves the water absorbing performance of the resin. When the monomer is a self-crosslink type compound, an internal crosslinked structure may be formed without using a radical polymerizable crosslinking agent. If necessary, a compound having two or more functional groups reactive with a carboxyl group may be added.

Examples of the radical polymerizable crosslinking agent include compound having, in one molecule thereof, a plurality of unsaturated bonds such as N,N-methylenebis(meth)acrylamide,(poly)ethylene glycol di(meth)acrylate, (poly)propyleneglycol di(meth)acrylate, trimethylolpropane tri(meth)acrylate, trimethylolpropane di(meth)acrylate, glycerin (meth)acrylate, glycerin acrylate methacrylate, ethylene-oxide modified trimethylolpropane tri(meth)acrylate, pentaerythritol tetra(meth)acrylate, dipentaerythritol hexa(meth)acrylate, triallyl cyanurate, triallyl isocyanurate, triallyl phosphate, triallylamine, and poly(meth)allyloxyalkane; compounds having, in one molecule thereof, a plurality of epoxy groups such as (poly)ethylene glycol diglycidyl ether and glycerol diglycidyl ether; and glycidyl (meth)acrylate. These radical polymerizable crosslinking agents may be used either singly or in combination of two or more thereof.

Examples of the compound having two or more functional groups reactive with a carboxyl group (carboxylic acid reactive crosslinking agent) include glycidyl ether compounds such as ethylene glycol diglycidyl ether, trimethylolpropane triglycidyl ether, (poly)glycerin polyglycidyl ether, diglycerin polyglycidyl ether, and propylene glycol diglycidyl ether; polyvalent alcohols such as (poly)glycerin, (poly)ethylene glycol, (poly)propylene glycol, 1,3-propanediol, polyoxyethylene glycol, triethylene glycol, tetraethylene glycol, 1,6-hexanediol, trimethylolpropane, diethanolamine, triethanolamine, polyoxypropylene, oxyethylene oxypropylene block copolymer, pentaerythritol, and sorbitol; polyvalent amines such as ethylenediamine, diethylenediamine, polyethyleneimine, and hexamethylenediamine; polyvalent aziridine compounds such as 2,2-bishydroxymethylbutanol-tris(3-(1-aziridinyl)propionate), various alkylene carbonate compounds such as 1,3-dioxolan-2-one, 4-methyl-1,3-dioxolan-2-one, and 4,6-dimethyl-1,3-doxolan-2-one; various polyvalent aldehyde compounds such as glyoxal; polyvalent oxazoline compounds such as 2,4-tolylene diisocyanate; haloepoxy compounds such as epichlorohydrin; and polyvalent ions such as zinc, calcium, magnesium, and aluminum.

One or more carboxylic acid reactive crosslinking agents selected from the group consisting of polyhydric alcohols, polyvalent glycidyl compounds, polyvalent amines, and alkylene carbonates is preferred.

The content of the carboxylic acid reactive crosslinking agent in a raw material solution for polymerization is preferably from 0 to 20 mol% relative to the total amount in moles of the monomers (the unsaturated carboxylic acid ammonium salt, the alkali metal salt of an unsaturated carboxylic acid, the unsaturated carboxylic acid, and the other monomer) and the radical polymerizable crosslinking agent. As in the water absorption theory of Flory, a resin having a lower crosslink density exhibits a higher water absorption ratio so that use of the crosslinking agent in a small amount is preferred. The content is preferably from 0 to 20 mol%, more preferably from 0 to 5 mol%, still more preferably from 0 to 0.09 mol%. Too large content of the carboxylic acid reactive crosslinking agent is not preferred because the gel thus obtained becomes hard and a water absorption ratio decreases drastically. The gel hardness can be controlled by combination use of a radical polymerizable crosslinking agent and the carboxylic acid reactive crosslinking agent. Accordingly, when the radical polymerizable crosslinking agent is used in a small amount within a range of from 0 to 0.09 mol% based on the total amount in moles of the monomers (the unsaturated carboxylic acid ammonium salt, the alkali metal salt of an unsaturated carboxylic acid, the unsaturated carboxylic acid, and the other monomer) and the radical polymerizable crosslinking agent, the carboxylic acid reactive crosslinking agent is used preferably within a range of from 0 to 5 mol%, more preferably within a range of from 0 to 3 mol% relative to the total amount in moles.

A foaming agent, a chain transfer agent, a chelating agent, and the like may be added as needed, in addition to the monomers and internal crosslinking agent.

The monomer concentration in the aqueous monomer solution at the time of initiation of polymerization is preferably 40 wt.% or greater and not greater than the solubility of the monomers in water. For example, the ammonium acrylate concentration is preferably from 45 to 80 wt.%, more preferably from 50 to 70 wt.%.

A higher monomer concentration is apt to accelerate the self crosslinking reaction so that it can reduce the using amount of the internal crosslinking agent necessary for insolubilization and raise a water absorption ratio of the water absorbing resin thus obtained.

When the monomer concentration is 40 wt.% or greater, a water-insoluble water absorbing resin can be produced using an internal crosslinking agent in an amount small enough to have substantially no adverse effect on the water retention property of the resin.

In addition, in solvent separation which will be described later, a higher monomer concentration is preferred because it facilitates filter separation between a hydrous gel thus formed and the solvent and enables employment of a simple process. Gels having a high water content are, on the other hand, tacky and when they are subjected to filter separation, the gels adhere firmly and integrate together. In this case, it is possible to collect the gel after evaporating the solvent and reducing its water content by azeotropic dehydration simultaneously.

Polymerization of the aqueous monomer solution may be performed after the total amount of the monomers is suspended in an organic solvent or while adding them to the organic solvent as needed.

In the present invention, a nonionic surfactant exist in an organic solvent.

The nonionic surfactant may be added to the organic solvent in advance or may be added thereto as needed during the polymerization step.

As the nonionic surfactant, those having an HLB of from 4 to 12 are preferred. When the organic solvent contains the nonionic surfactant having an HLB within the above-described range, a polymerization reaction solution forms a stable emulsion and large particles can be formed more stably. Surfactants having an HLB from 5 to 10 are more preferred. Specific examples of the nonionic surfactant having an HLB of from 4 to 12 include sorbitol fatty acid esters, sorbitol fatty acid ester ethers, sorbitan fatty acid esters, and sorbitan fatty acid ester ethers. Of these, sorbitan fatty acid esters and sorbitan fatty acid ester ethers are preferred. Of these, sorbitan monostearate, sorbitan monolaurate, and oxyethylene sorbitan monostearate ether having an HLB of from 5 to 10 are more preferred. Sorbitan monostearate is still more preferred.

The HLB in the first embodiment of the present invention means Griffin's HLB as described in Shin Kaimenkasseizai Nyumon published by Sanyo Kasei Kogyo. The calculating formula of the HLB is defined as follows:

HLB of nonionic surfactant = (molecular weight of hydrophilic group portion)ö(molecular weight of surfactant)x20

The appropriate using amount of the surfactant ranges from 0.1 to 15 wt.%, preferably from 0.2 to 5 wt.%. Too small using amounts are not effective for maintaining a stable emulsion state, while using amounts of 15 wt.% or greater cannot bring about satisfactory results proportion to the using amount.

In the first embodiment of the present invention, any organic solvent that is separated into two layers after having been mixed with an equal amount of water and left at rest can be used. There is no limitation on the type or amount of the functional group, and constituent atoms insofar as the organic solvent does not severely inhibit the radical polymerization reaction of the raw material monomers.

As the process solvent, solvents having a small evaporative latent heat, having good separability from water, and not chemically reacting easily with the surfactant are usually preferred.

More specifically, a hydrocarbon solvent is preferred, with an aliphatic hydrocarbon solvent being more preferred and a saturated aliphatic hydrocarbon solvent being still more preferred. The saturated aliphatic hydrocarbon solvent may have any of a linear structure, a branched structure, or a cyclic structure. A compound having, in one molecule thereof, a plurality of structures selected from a linear structure, a branched structure and a cyclic structure can also be used.

Specific examples of the saturated aliphatic hydrocarbon solvent include saturated aliphatic hydrocarbon solvents having a cyclic structure such as cyclopentane, cyclohexane, methylcyclopentane, methylcyclohexane, and cyclooctane; and saturated aliphatic hydrocarbons having a linear structure such as n-pentane, n-hexane, n-heptane, n-octane, and ligroin. From the viewpoint of stability of the emulsion thus obtained and various physical properties of the solvent such as boiling point and specific gravity, cyclopentane, cyclohexane, cyclooctane, n-pentane, and n-hexane are preferred among them, with cyclohexane being more preferred.

The polymerization initiation method is not particularly limited and polymerization may be initiated by the use of a radical polymerization initiator or exposure to radiation or electron beam, or ultraviolet polymerization may be initiated with a photosensitizer.

Examples of the initiator used for such radical polymerization include known initiators such as persulfates, e.g., potassium persulfate, ammonium persulfate, and sodium persulfate; hydrogen peroxide; and organic peroxides, e.g., cumene hydroperoxide, t-butylhydroperoxide, and peracetic acid.

When an oxidative radical polymerization initiator is used, a reducing agent such as L-ascorbic acid or sodium hydroxymethanesulfinate dehydrate ("Rongalit", trade name; product of Wako Pure Chemical Industry) may be used in combination.

These initiators may be used either singly or in combination of two or more.

A deoxygenetion operation for the monomer solution is carried out preferably in advance before the polymerization is initiated. Specifically, dissolved oxygen is removed, for example, by bubbling with an inert gas for an adequate period of time.

The atmosphere in a reactor is preferably purged with an inert gas such as nitrogen or helium.

The pressure in the polymerization reactor may be any of reduced pressure, normal pressure, or applied pressure.

The polymerization initiation temperature is usually from 0 to 100°C, but no particular limitation is imposed on it. The polymerization initiation temperature is preferably from 10 to 50°C. The temperature during polymerization is generally equal to the initiation temperature and is from 0 to 100°C, preferably from 40 to 80°C. The temperature in the reactor during the polymerization reaction may depend on the situation or may be controlled by cooling or heating from the outside. Alternatively, the reaction temperature may be controlled by the boiling point of the solvent. The control of the reaction temperature by the boiling point of the solvent is preferably conducted by adjusting the pressure of a gas phase, thereby controlling the boiling point. Polymerization control by changing the reaction temperature during from the polymerization initiation to the polymerization completion is preferred. For example, it is very preferred to suppress the temperature at the initial period of the reaction to a relatively low temperature in order to prevent a runaway reaction and then raise the polymerization degree at the end period of the reaction to reduce the remaining monomers.

The type or amount of the surfactant, a ratio of the aqueous monomer solution phase to the organic solvent phase, and the magnitude of a stirring power in the polymerization step have a large influence on the primary particle size of agglomerated particles thus formed.

(Agglomeration step)

In the agglomeration step after completion of the polymerization reaction, the primary particles are agglomerated by using a water soluble solvent. The term "water soluble solvent" as used herein means an organic solvent having a solubility in water of 1 wt.% or greater.

More specifically, the primary particles are agglomerated preferably in the presence of the water soluble solvent. Agglomeration in the presence of the water soluble solvent is preferably achieved by mixing the emulsion solution after polymerization with the water soluble solvent. The water soluble solvent may be added to the emulsion; the emulsion may be added to the water soluble solvent; or the emulsion and the water soluble solvent may be added to a reaction vessel simultaneously. Addition of the water soluble solvent to the emulsion solution after polymerization is a simple and easy process. Addition of the water soluble solvent to the stirred emulsion solution is employed as a preferred method. When the emulsion and the water soluble solvent are mixed, a stabilizing effect of the surfactant serving to maintain the emulsion state is destroyed and the primary particles are therefore agglomerated. For destroying the stabilizing effect of the surfactant, the water soluble solvent to be mixed must have a solubility in water of 1 wt.% or greater, preferably 5 wt.% or greater, more preferably 10 wt.% or greater.

Examples of the water soluble solvent include ketones such as acetone and methyl ethyl ketone; nitriles such as acetonitrile and propionitrile; amides such as dimethylformamide and N,N-dimethylacetamide; esters such as ethyl acetate, methyl acetate, and methyl propionate; ethers such as tetrahydrofuran, diethyl ether, and methyl ethyl ether; monoalcohols such as methyl alcohol, ethyl alcohol, n-propyl alcohol, isopropyl alcohol, and cyclohexanol; and polyvalent alcohols such as ethylene glycol, propylene glycol, polyethylene glycol, propione glycol, glycerin, 1,2-cyclohexanediol, and 1,6-hexanediol. Of these, monoalcohols and polyvalent alcohols are preferred.

These water soluble solvents may be added either singly or in combination, but use of two or more water solvents is preferred. More preferably, two or more water soluble solvents including the polyvalent alcohol are used. Use of the polyvalent alcohol having two or more alcohol groups as the water soluble solvent is preferred because it is highly effective for reducing a water soluble component generated during the agglomeration step. When two or more of the water soluble solvents are used, they may be added simultaneously or individually.

A water soluble solvent using a monoalcohol and a polyvalent alcohol in combination is preferred. As the monoalcohol, methyl alcohol, ethyl alcohol, or isopropyl alcohol is preferred, while as the polyvalent alcohol, propylene glycol, glycerin, or ethylene glycol is preferred. Combination of ethyl alcohol and glycerin or combination of isopropyl alcohol and glycerin is most preferred.

The amount of the water soluble solvent is not limited, but excessive amounts of the water soluble solvent may decrease a water absorption ratio of the resulting water absorbing resin particle agglomerates. Accordingly, the amount of the water soluble solvent is preferably from 0.1 to 20 wt.%, more preferably from 1 to 10 wt.% based on the solid content (that is, water soluble resin particles) in the emulsion.

The temperature at the time of addition of the water soluble solvent is not particularly limited insofar as it maintains the emulsion state. Addition may be performed without changing the polymerization temperature or may be performed after having increased the temperature. It is also possible to add the solvent after cooling to approximately room temperature. The temperature at the time of the addition is preferably from 25 to 120°C, more preferably from 50 to 110°C, still more preferably from 65 to 100°C.

After the agglomeration step, a step of azeotropic hydration with the solvent may be comprised to reduce a water content of the particle agglomerate gel. The conditions of pressure or temperature for azeotropic hydration are not limited particularly.

The particle size of the agglomerated secondary particles can be controlled and a desired particle size can be achieved by controlling the amount of the water soluble solvent or the magnitude of the stirring power. In the first embodiment of the present invention, the particle size of the secondary particles is not particularly limited. Particles with a small particle size are not used in the field of hygiene materials where water absorbing resins are most frequently used because dust generated from small particles causes a problem. Particles with an excessively large particle size are also not used because a water absorption rate is low. With the foregoing in view, the particle size from 100 to 5000 $\mu$m is preferred, with a particle size from 300 to 3000 $\mu$m being particularly preferred.

(Fusion bonding step)

In order to increase the bonding power of the agglomerated particles, it is effective to employ a step of fusing bonding particles by maintaining the temperature of the emulsion at 40°C or greater after formation of the agglomerates, that is, after completion of the addition of the water soluble solvent in order to enhance the bonding strength of the agglomerated particles. The reason why such heat treatment is effective has not been elucidated but it is presumed that free polymer chains of the contacted particles or segments thereof diffuse mutually and so-called self adhesion proceeds. Heating is therefore performed preferably at a temperature equal to or greater than the glass transition point of the hydrous particle agglomerate gel which has been agglomerated in order to promote mutual diffusion of the polymer chain. The glass transition point of the gel changes depending on the water content, neutralization ratio, or kind of the neutral salt of the gel, and the heating temperature is preferably from 40 to 200°C, more preferably from 60 to 180°C, still more preferably from 60 to 150°C. Heating time is preferably from 1 to 120 minutes. The temperature and time are not limited particularly insofar as they are sufficient for fusing the gel and do not deteriorate the performance of the product. In order to raise the heating temperature, application of pressure is effective, and using a solvent different from the one that is used for polymerization is also effective.

Although the bonding strength of the agglomerated particles is not particularly limited, the strength is preferably high in consideration of the handling of the produced resin. The bonding strength is preferably 1N or greater as measured by a Kiya type strength meter which will be described later.

(Collection step)

After formation of the particle agglomerate gel, the hydrous gel thus formed is collected. The separation between the solvent and the hydrous gel is performed, for example, by filtration, centrifugal separation, or removal of the solvent by heating, and any of these methods is usable.

(Drying step)

The drying method of the particle agglomerate gel is not particularly limited and vacuum drying or hot air drying is generally employed. The drying temperature is preferably from 70 to 180°C, more preferably from 90 to 140°C. The drying step may be performed by elevating the temperature in multiple-stage. Too low drying temperatures are not economical because it takes much time for drying, while too high drying temperatures may cause decomposition of the water absorbing resin and therefore deteriorate the water absorption performance.

(Heating step)

When an ammonium salt is used as the water absorbing resin, the ammonia neutralization ratio can be controlled to a desired ratio by heat treating the water absorbing resin after the above-described drying to release ammonia. The ammonia are released free from the resin surface so that a neutralization ratio of the water absorbing resin on the outer surface of the water absorbing resin particle agglomerates can be reduced. Such a heating step can therefore be utilized for the production of the water absorbing resin particle agglomerates of the second embodiment of the present invention. At the same time, the water soluble component amount can be reduced by reacting the water soluble solvent such as polyvalent alcohol added during the agglomeration step with a functional group in a low molecular weight polymer which will be a water soluble component, thereby converting the low molecular weight polymer into a high molecular weight one. The heating step may be performed while making the water absorbing resin after the drying step to coexist with a nonwoven fabric or pulp in a contacted, adhered, or attached state or it may be performed for only the water absorbing resin.
The heating temperature is preferably from 130 to 250°C, more preferably from 150 to 200°C. Heating is conducted preferably at a temperature higher by from 10 to 150°C, more preferably from 30 to 100°C than the drying temperature from the viewpoint of the distribution structure of a neutralization ratio in the resin and water absorption performance.
The heating time is preferably from 0.5 minute to 5 hours, more preferably from 2 to 60 minutes, still more preferably from 3 to 15 minutes.
The heat treatment atmosphere is not limited particularly, and heat treatment is performed preferably in a nitrogen atmosphere.
It is also within the scope of the present invention to perform a so-called surface crosslinking by impregnating the water absorbing resin after the drying step with a compound having two or more functional groups reactive with the carboxyl group and causing a crosslinking reaction by heating.
Examples of the second embodiment of the present invention will next be described specifically.
The water absorbing resin particle agglomerates according to the second embodiment of the present invention are secondary particles obtained by the agglomeration of primary particles.
First, primary particles constituting the water absorbing resin particle agglomerates of the second embodiment of the present invention are described.
The primary particles in the second embodiment of the present invention are made of a water absorbing resin in which 50 mol% or greater of the repeating units in the polymer molecular chain are carboxyl group-containing units and at least a portion of the carboxyl groups of the carboxyl-containing units has been neutralized with at least one base selected from alkali metals, amines and ammonia.
No limitation is imposed on the manufacturing method of the primary particles insofar as it can produce the water absorbing resin particle agglomerates of the second embodiment of the present invention. A known process is usable, and a manufacturing method of the first embodiment of the present invention is suited for use.
The primary particles may be in either a spherical form or an infinite form.
The particle size of the primary particles is not limited insofar as it permits to produce second particles having a desired particle size after agglomeration. The primary particles have an average particle size of preferably from 30 to 1000 $\mu$m. In consideration of the water absorption rate of the secondary particles, primary particles having a relatively small diameter is preferred. The average particle size is adjusted to preferably from 30 to 500 $\mu$m, more preferably from 30 to 300 $\mu$m.
It is also possible to use, without problems, primary particles having a predetermined latitude in particle size or having a plurality of peaks in its distribution.
In the present invention, the term "average particle size" means a value determined by the method described below.

The particles are sieved through sieves having openings of 20 $\mu$m, 40 $\mu$m, 75 $\mu$m, 106 $\mu$m, 212 $\mu$m, 300 $\mu$m, 425 $\mu$m, 500 $\mu$m, 600 $\mu$m, 710 $\mu$m, 850 $\mu$m, 1000 $\mu$m, 1180 $\mu$m, 1400 $\mu$m, 1700 $\mu$m, 2000 $\mu$m, 4000 $\mu$m, and 5600 $\mu$m, respectively and an intermediate value between the opening of the sieve through which the particles can pass and the opening of the sieve through which the particles cannot pass is determined as a classification particle size of the particles. The particles have one of the classification particle sizes of 10 $\mu$m, 30 $\mu$m, 57.5 $\mu$m, 90.5 $\mu$m, 159 $\mu$m, 256 $\mu$m, 362.5 $\mu$m, 462.5 $\mu$m, 550 $\mu$m, 655 $\mu$m, 780 $\mu$m, 925 $\mu$m, 1090 $\mu$m, 1290 $\mu$m, 1550 $\mu$m, 1850 $\mu$m, 3000 $\mu$m, 4800 $\mu$m, and 6000 $\mu$m. Particles which can pass through a sieve of 20 $\mu$m is determined to have a classification particle size of 10 $\mu$m, while particles which remain on the sieve of 5600 $\mu$m is determined to have a classification particle size of 6000 $\mu$m. A value (which will hereinafter be called "classification particle size weight value") is obtained by multiplying each classification particle size with a weight percentage (%) of particles belonging to the classification particle size based on a total weight of the particles. Then, the sum of the classification particle size weight values of all the classification particle sizes is calculated and a value obtained by dividing the sum with 100 is determined as an average particle size of the particles.

In the water absorbing resin constituting the primary particles, 50 mol% or greater of the repeating units in the polymer molecular chain are carboxyl group-containing units. From the viewpoint of water absorbing performance, 80 mol% or greater, more preferably 90 mol% or greater of the repeating units are carboxyl group-containing units.

No limitation is imposed on the carboxyl group-containing monomer from which the carboxyl group-containing unit in the water absorbing resin constituting the primary particles is derived from and specific examples of it include acrylic acid, methacrylic acid, itaconic acid, maleic acid, crotonic acid, fumaric acid, sorbic acid, and cinnamic acid, and anhydrides or neutral salts thereof.

The carboxyl group in the water absorbing resin constituting the primary particles is partially neutralized and the neutralizing base is at least one of alkali metals such as sodium, potassium, and lithium, amines and ammonia. The neutralizing base preferably contains ammonia.

From the viewpoint of enhancing a water absorption ratio of the water absorbing resin agglomerates, preferably 50 mol% or greater of the carboxyl neutralized salts in the water absorbing resin constituting the primary particles are ammonium salts. More preferably 70 mol% or greater, still more preferably 100 mol% are ammonium salts.

No limitation is imposed on the monomer components, other than the carboxyl group-containing monomer, of the water absorbing resin constituting the primary particles and specific examples mainly include monofunctional unsaturated monomers such as acid-group-containing hydrophilic monofunctional unsaturated monomers such as vinylsulfonic acid, styrenesulfonic acid, 2-(meth)acrylamido-2-methylpropanesulfonic acid, 2-(meth)acryloylethanesulfonic acid, and 2-(meth)acryloylpropanesulfonic acid, and salts thereof; amide-containing hydrophilic monofunctional unsaturated monomers such as acrylamide, methacrylamide, N-ethyl (meth)acrylamide, N-n-propyl (meth)acrylamide, N-isopropyl (meth)acrylamide, and N,N-dimethyl (meth)acrylamide; esterified hydrophilic unsaturated monomers such as 2-hydroxyethyl (meth)acrylate, 2-hydroxypropyl (meth)acrylate, methoxypolyethylene glycol (meth)acrylate, and polyethylene glycol mono(meth)acrylate; N-atom-containing hydrophilic monofunctional unsaturated monomers typified by vinyl pyridine, N-vinylpyrrolidone, N-acryloylpiperidine, N-acryloylpyrrolidine, N,N-dimethylaminoethyl (meth)acrylate, N,N-diethylaminoethyl (meth)acrylate, N,N-dimethylaminopropyl (meth)acrylate, and N,N-dimethylaminoethyl (meth)acrylamide, and quaternary salts thereof; and hydrophobic monofunctional unsaturated monomers such as styrene, vinyl chloride, butadiene, isobutene, ethylene, propylene, and alkyl (meth)acrylate.

Of these, (meth)acrylic acid (salt thereof), 2-(meth)acryloylethanesulfonic acid (salt thereof), 2-(meth)acrylamido-2-methylpropanesulfonic acid (salt thereof), methoxypolyethylene glycol (meth)acrylate, N,N-dimethylaminoethyl (meth)acrylate, and (meth)acrylamide are preferred.

The water absorbing resin constituting the primary particles can further contain a deodorant, an antibacterial agent, a perfume, various inorganic powders, a foaming agent, a pigment, a dye, hydrophilic short fibers, a fertilizer, an oxidizing agent, a reducing agent, water, salts, or the like to impart the resin with various functions. The "water absorbing resin" in the second embodiment of the present invention includes a water absorbing resin composition containing such an additive.

The water absorbing resin particle agglomerates according to the second embodiment of the present invention will hereinafter be described. The water absorbing water resin particle agglomerates according to the second embodiment of the present invention are secondary particles obtained by agglomerating the primary particles. No limitation is imposed on the method of agglomerating the primary particles, and the manufacturing method of the agglomerates in the first embodiment of the present invention is suited.

The water absorbing resin particle agglomerates according to the second embodiment of the present invention have, on the outer surface thereof, a portion having a neutralization ratio of carboxyl groups in the water absorbing resin not greater than 40 mol% and have, in the inside, a portion having a neutralization ratio of carboxyl groups in the water absorbing resin equal to or greater than 50 mol%.

The term "neutralization ratio of carboxyl groups" as used herein means a molar percentage of neutralized carboxyl groups relative to all the carboxyl groups in the water absorbing resin; the term "outer surface" of the water absorbing resin particle agglomerates means a portion of the agglomerates which is exposed to outside. The neutralization ratio of the water absorbing resin inside of the water absorbing resin particle agglomerates is preferably 60 mol% or greater, more preferably 70 mol% or greater. The neutralization ratio of the water absorbing resin on the outer surface of the water absorbing resin particle agglomerates is preferably 35 mol% or less, more preferably 30 mol% or less.

The neutralization ratio inside the water absorbing resin particle agglomerates is preferably as high as possible because such agglomerates show a high water retention property (water absorption ratio) as a whole. The neutralization ratio on the outer surface of the water absorbing resin particle agglomerates is preferably as low as possible because such agglomerates do not easily cause a gel blocking phenomenon which is a so-called *Mamako* phenomenon.

No limitation is imposed on a method of adjusting the neutralization ratios on the outer surface and inside of the water absorbing resin particle agglomerates to values specified in the second embodiment of the present invention. The neutralization ratio can be reduced, for example, by forming the agglomerates of primary particles made of a water absorbing resin having a high neutralization ratio and heat treating the agglomerates to release the neutral salt from the outer surface.

The neutralization ratio can also be reduced by impregnating the dried water absorbing resin particle agglomerates with a compound having two or more functional groups reactive to a carboxyl group and causing a crosslinking reaction by heating, that is, carrying out surface crosslinking treatment.

In particular, the method of reducing a neutralization ratio by heating to release the neutral salt from the outer surface is preferred, because in addition to the benefit that it is simple, it can reduce both the neutralization ratio on the outer surface of the agglomerates and that on the outer surface of the primary particles that present inside of the agglomerates, thereby preventing gel blocking between the primary particles which will otherwise occur inside the agglomerates.

Heating conditions are not limited and they are set as needed so that the neutralization ratio inside the agglomerates falls within a range of 60 mol% or greater and the neutralization ratio on the outer surface layer fall within a range of 40 mol% or less.

Described specifically, such a heat treatment may be performed while making the dried water absorbing resin particle agglomerates to contact, bond or attach to a nonwoven fabric or pulp or it may be performed only for the water absorbing resin particle agglomerates.

The heating temperature is preferably from 100 to 250°C, more preferably from 120 to 200°C. Heating is conducted preferably at a temperature higher than the drying temperature at the time of forming of the agglomerates by from 10 to 150°C, more preferably from 30 to 100°C from the viewpoint of the distribution pattern of a neutralization ratio in the resin and water absorption performance. The heating time is preferably from 0.5 minute to 5 hours, more preferably from 2 to 60 minutes, still more preferably from 3 to 15 minutes.

The atmosphere during the heat treatment is not particularly limited, and the treatment is performed preferably in a nitrogen atmosphere.

The carboxyl group neutralization ratio of the water absorbing resin in the present invention can be measured by the microscopic ATR method which is one of infrared absorption analysis methods. The neutralization ratio on the outer surface of the agglomerates can be determined by directly measuring the outer surface of the agglomerates by the microscopic ATR. The neutralization ratio inside the agglomerate can be determined by cutting the agglomerates, for example, with a ultramicrotome ("ULTRACUT N", product or Reichert) to expose the inside portion and then measuring the neutralization ratio by the microscopic ATR method. As a measurement apparatus, "FTS-575" product of Bio-Rad, for example, can be used.

In order to measure the carboxyl group neutralization ratio by the microscopic ATR method, 1695 cm$^{-1}$ (v C=O of carboxylic acids, base line from 1774 to 1616 cm$^{-1}$) and 1558 cm$^{-1}$ (v COO- of carboxylates, base line from 1616 to 1500 cm$^{-1}$), for example, can be used as an index for specifying a composition ratio of carboxylic acids and carboxylates and the peak area ratio (1695/1558 cm$^{-1}$) is measured.

Alternatively, measurement is conducted using samples which have known carboxyl group neutralization ratios, for example, partially crosslinked polyacrylic acids in which 10 mol%, 30 mol%, 50 mol%, 70 mol%, 90 mol% and 100 mol% of all the carboxylic acids have been neutralized with ammonia as standard samples and the carboxyl group neutralization ratio can be determined based on a calibration curve created thereby.

The shape of the water absorbing resin particle agglomerates according to the second embodiment of the present invention is not particularly limited.

In the field of hygiene materials, the water absorbing rein particle agglomerates are sometimes mixed with pulp and used as a water absorbing composite so that they are preferably in the form of spherical particles or infinite form particles from the viewpoint of handling convenience such as ease of mixing with pulp. The water absorbing resin particle agglomerates have an average particle size of preferably from 100 to 5000 μm, more preferably from 550 to 2100 μm, most preferably from 780 to 1550 μm. The agglomerates having an excessively small particle size become fine dusts and are apt to scatter, which cause a problem during use. The agglomerates having an excessively large particle size cause, on the other hand, problems such as reduction in water absorption rate and uneven distribution of the water absorbing resin particle agglomerates in absorbent articles.

Particularly when the agglomerates are used for a water absorbing composite, it is preferred to use the agglomerates having the above-described classification particle size as large as approximately 550 μm in order to increase the water absorption ratio per unit area of the absorbing composite.

The bonding strength of the water absorbing resin particle agglomerates is not particularly limited. The agglomerates having a high strength are preferred in view of the handling of the resin thus produced. It is preferably 1 N or greater, more preferably 5N as measured by a Kiya-type strength meter which will be described later.

Next, application of the water absorbing resin particle agglomerates produced by the manufacturing method according to the first embodiment of the present invention and the water absorbing resin particle agglomerates according to the second embodiment of the present invention to body-fluid absorbing articles will be described.

The term "body-fluid absorbing articles" as used herein means any body-fluid absorbing articles made of liquid permeable sheets and water absorbing absorbent placed therebetween and having an ability of absorbing body fluids. The body fluid to be absorbed is not limited and examples include urine, menstrual blood, mother's milk, soft stool and the like. There are also no particular limitations on the shape of the article, and desirable examples include pads, tapes, and pants. Specific examples of the body-fluid absorbing articles include diapers, sanitary napkins, incontinence pads, and lactation pads.

The body-fluid absorbing articles of the present invention have, as the absorbent thereof, the water absorbing resin particle agglomerates produced by the manufacturing method of the first embodiment of the present invention and/or the water absorbing resin particle agglomerates according to the second embodiment of the present invention.

The constitution of the absorbent is not limited, and examples of it include a mixture of a fibrous substance such as pulp and the water absorbing resin particle agglomerates, and the water absorbing resin particle agglomerates fixed onto a base material.

Examples

Manufacturing examples will hereinafter be described, but the present invention is not limited to these examples. In the manufacturing examples, measurement and evaluation were performed in accordance with the following methods.

(Measurement of a carboxyl group neutralization ratio on the outer surface and inside of a water absorbing resin particle agglomerates)

(1) Measuring apparatus

"FTS-575", product of Bi-Rad Company was used as a measuring apparatus.

(2) Measurement conditions

The microscopic ATR spectroscopy (crystal plate of Ge, single reflection) was employed and measurement was conducted under the conditions of: air as a background, measurement at normal temperature, aperture of $50 \times 50$ μm, and integration numbers of 100 times.

From the spectrum data obtained by the measurement, a peak area ratio (1695/1558 cm$^{-1}$) of 1695 cm$^{-1}$ (ν C=O of carboxylic acids, base line, from 1774 to 1616 cm$^{-1}$) to 1558 cm$^{-1}$ (ν COO- of carboxylates, base line, from 1616 to 1500 cm$^{-1}$) is determined.

(3) Preparation of calibration curve

Partially crosslinked polyacrylic acids prepared by neutralizing 10 mol%, 30 mol%, 50 mol%, 70 mol%, 90 mol% and 100 mol% of all the carboxylic acids with ammonia were used as the samples for preparing the calibration curve. Each of the samples for preparing the calibration curve was cut and the central portion of the sample was measured five times/sample by the microscopic ATR spectroscopy. The calibration curve (quintic polynomial approximation curve) was prepared based on the average of a -COOH/-COO peak area ratio.

Cutting was performed using a ultramicrotome ("ULTRACUT N", product of Reichert Company).

(4) Measurement of sample

Measurement was performed in a similar manner to that employed for the sample for preparing a calibration curve. As measurement samples, samples with a particle size of from 300 to 700 μm were used. The outer surface of the water absorbing resin particle agglomerates was measured by the ATR spectroscopy directly, while the inside of the agglomerates was measured by the ATR spectroscopy after cutting the inside of the agglomerates by using a ultramicrotome. Measurement of the outer surface was performed three times/sample and the minimum value was used as a measurement result. Measurement of the inside was performed five times/sample and the maximum value was used as a measurement result.

(Measurement of water retention property of water absorbing resin (primary particles and primary particle agglomerates); Tea bag method)

Sample A (g) (about 0.5 g) was filled uniformly in a tea-bag type bag (7×9 cm) made of a nonwoven fabric, and immersed in 500 cc of physiological saline of 25°C until it reached equilibrium swelling. After a predetermined time, the tea-bag type bag was taken out, and water was drained off naturally for 10 minutes. The weight (B) (g) of the tea-bag type bag was measured. A similar operation was performed as a blank by using a tea-bag type bag without adding the sample thereto. Weight C (g) was measured and a water absorption ratio was determined in accordance with the following equation.
Water absorption ratio (g/g) = (B (g) - C (g))/A (g)

(Measurement of initial water absorption rate of water absorbing resin (primary particles and primary particle agglomerates)

Sample A' (g) (about 0.2 g) was put uniformly in a tea-bag type bag (7×9 cm) made of a nonwoven fabric and immersed in 500 cc of physiological saline having a liquid temperature of 25°C for one minute. The tea-bag type bag was then taken out and set on a centrifugal separator. Centrifugal separation was performed while setting the conditions of the centrifugal separator as follows: at 1500 rpm for 3 minutes. The weight B' (g) of the tea-bag type bag after centrifugal separation was weighed. As a blank, weight C' (g) was measured in the same way using a tea-bag type bag without putting a sample therein. The water absorption ratio was determined based on the following equation and it was determined as an initial water absorption rate. Initial water absorption rate = water absorption ratio (g/g) = (B' (g) - C' (g))/A' (g)

(Measurement of bonding strength of water absorbing resin particle agglomerates)

The bonding strength of the water absorbing resin particle agglomerates was measured using a Kiya type digital hardness meter "KHT-20N", product of Fujiwara Seisakujo. The particle agglomerates provided for the measurement had a diameter of 2 mm. The bonding strength was measured ten times and average of the measured values except for the maximum and minimum values was determined.

(Measurement of water soluble component amount of water absorbing resin)

After 0.500 g of a water absorbing resin was dispersed in 1000 ml of deionized water and the resulting dispersion was stirred at 23°C for 16 hours, the reaction mixture was filtered through a filter paper.
Next, 50 g of the filtrate was weighed in a 100-ml beaker and 1 ml of a 0.1 mol/liter aqueous solution of sodium hydroxide, 10.00 ml of an aqueous N/200-methylglycol chitosan solution, and 4 drops of a 0.1 wt.% aqueous solution of toluidine blue were added.
The solution in the beaker was subjected to colloid titration with an aqueous N/400-potassium polyvinylsulfate solution and a titration amount at the time when the color of the solution changed from blue to reddish violet was determined to be a titration amount A (ml) of terminal point of the titration.
The same operation except that 50 g of deionized water was used instead of 50 g of the filtrate was performed to determine a titration amount B (ml) for blank titration.
Based on these titration amounts A (ml) and B (ml) and a neutralization ratio C (mol%) of acrylic acid provided for the production of a water absorbing resin, a water soluble component amount (wt.%) of the water absorbing resin was calculated in accordance with the following equation: Water soluble component amount (wt.%) = (B-A)x0.01 x(72x (100-C)+89 × C)/100

(Measurement of absorption ratio of absorbent)

An absorbent was cut into a circle having a diameter of 59.5 mm and its weight A" (g) was measured. A wire was penetrated through a point 1 cm inside from the circumference of the absorbent. The absorbent and the wire were both immersed in 500 cc of physiological saline having a liquid temperature of 25°C. Three hours later, the absorbent was taken out from the physiological saline and it was suspended for 10 minutes while preventing it from contacting with anything else. After draining, the wire was removed and the total weight of the water containing absorbent and water attached thereto B"(g) was measured. The absorption ratio of the absorbent was determined in accordance with the following equation:

$$\text{Absorption ratio (g/g) of absorbent} = B''\,(g)/A''\,(g)$$

(Measurement of initial water absorption speed of absorbent)

An absorbent was cut into a circle having a diameter of 59.5 mm and its weight A''' (g) was measured. A wire was penetrated through the circle 1 cm inside from the circumference of the absorbent. The absorbent and the wire were both immersed for one minute in 500 cc of physiological saline having a liquid temperature of 25°C. The absorbent was taken out from the physiological saline and remove the wire, and then the absorbent was set in a centrifugal separator. Centrifugal separation was performed under the conditions of 1500 rpm and 3 minutes. The weight B''' (g) of the absorbent after the centrifugal separation was measured. The water absorption ratio of the absorbent was determined in accordance with the following equation and it was determined as an initial water absorption rate.

$$\text{Initial water absorption speed of absorbent} = \text{absorption ratio (g/g) of absorbent}$$
$$= B'''\,(g)/A'''\,(g)$$

Manufacturing Examples A1 to A13 of water absorbing resin particle agglomerates/water absorbing resin particles will hereinafter be described. Detailed manufacturing conditions and physical properties of the resulting water absorbing resin particle agglomerates/water absorbing resin particles are shown in Table 2.

(Manufacturing Example A1)

After 211.75 g of acrylic acid obtained by distilling and purifying special-grade acrylic acid produced by Wako Pure Chemicals was weighed in a 500-ml flask, 188.50 g of 26.5 wt.% of aqueous ammonia was added dropwise thereto under stirring while cooling to yield 400.25 g of an aqueous solution of ammonium acrylate having a neutralization ratio of 100 mol%.
As a radical polymerizable crosslinking agent, 0.026 g of N,N'-methylenebisacrylamide dissolved in 0.5 g of water was added to the aqueous solution. The resulting mixture was dissolved by stirring. 0.1081 g of ammonium persulfate dissolved in 0.5 g of water was also added as a polymerization initiator in the same way.
A 2-L separable flask purged with nitrogen in advance and equipped with a reflux condenser was charged with 400 g of cyclohexane and 1.91 g of sorbitan monostearate as a surfactant. After stirring at room temperature to dissolve them, the aqueous solution of ammonium acrylate obtained above was added to the resulting solution. While feeding nitrogen, stirring was performed sufficiently at 200 rpm to obtain a suspension. Then, polymerization was initiated while reducing the pressure inside the reactor to 65 kPa and keeping the internal temperature at 60°C on a water bath of 60°C. The suspension was retained for 2 hours while keeping a stirring rate at 200rpm and an emulsion containing a hydrous gel was obtained.
The pressure was returned to normal while blowing nitrogen into the reactor and the temperature was raised to 75°C. The stirring rate was set at 300 rpm, then 16 g of isopropanol produced by Wako Pure Chemicals was added as an alcohol having a water solubility of 1 wt.% or greater over 5 minutes. After large particles were formed by agglomeration, the heating condition was maintained while stirring. Heating was continued for one hour.
The hydrous gel thus obtained was collected by filtration, vacuum dried at 100°C and then collected.
The primary particle agglomerates had an average particle size of 1200 $\mu$m and 6 wt.% of them had a particle size less than 300 $\mu$m. The water absorption ratio as measured by the tea bag method was 70.1 times.

(Manufacturing Example A2)

After 95.04 g of acrylic acid obtained by distilling and purifying special-grade acrylic acid produced by Wako Pure Chemicals was weighed in a 300-ml flask, 89.96 g of 25 wt.% aqueous ammonia was added dropwise under stirring while cooling to yield 185.00 g of an aqueous solution of ammonium acrylate having a neutralization ratio of 100 mol%.

To the resulting aqueous solution was added 0.0027 g of N,N'-methylenebisacrylamide dissolved in 0.5 g of water. The resulting mixture was dissolved by stirring. 0,0920 g of ammonium persulfate dissolved in 0.5 g of water was also added in the same way.

A 2-L separable flask purged with nitrogen in advance and equipped with a reflux condenser was charged with 450 g of cyclohexane and 1.1125 g of sorbitan monostearate as a surfactant. After stirring at room temperature to dissolve them, the aqueous solution of ammonium acrylate obtained above was added to the resulting solution. While feeding nitrogen, stirring was performed sufficiently at 200 rpm to obtain a suspension. Then, polymerization was initiated while reducing the pressure inside the reactor to 65 kPa and keeping the internal temperature at 60°C with a water bath of 60°C. The suspension was retained for 2 hours while keeping a stirring rate maintained at 200 rpm and an emulsion containing a hydrous gel was obtained.

The pressure was returned to normal while blowing nitrogen into the reactor and the temperature was raised to 75°C. The stirring rate was set at 300 rpm, then 8.5 g of special-grade ethanol produced by Wako Pure Chemicals was added as an alcohol having a water solubility of 1 wt.% or greater over 5 minutes. After large particles were formed by agglomeration, the heating condition was maintained while stirring. Heating was continued for one hour.

The hydrous gel thus obtained was collected by filtration, vacuum dried at 100°C, and then collected. The first particle agglomerates had an average particle size of 1200 $\mu$m and 6 wt.% of them had a particle size less than 300$\mu$ m. The water absorption ratio as measured by the tea bag method was 65.5 times. The resin hardness was 6.5N.

(Manufacturing Example A3)

Ammonium acrylate was prepared by the hydrolysis of acrylonitrile in the following manner. The hydrolysis of acrylonitrile was performed in accordance with the process of Example 4 of Japanese Patent Laid-Open No. 2004-305062 with a biocatalyst prepared in accordance with the process of Example 1.

(Preparation of biocatalyst)

*Acinetobacter sp.* AK226 (FERM BP-08590) having a nitrilase activity was aerobically cultured at 30°C on a culture medium adjusted to pH 7, with an aqueous solution containing 0.1% of sodium chloride, 0.1% of potassium dihydrogen phosphate, 0.05 % of magnesium sulfate heptahydrate, 0.005% of iron sulfate heptahydrate, 0.005% of managanese sulfate pentahydrate, 0.1 % of ammonium sulfate, and 0.1% of potassium nitrate (each, weight%) by adding 0.5 wt.% acetonitrile as a nutrition source to the culture medium. The resulting culture medium was washed with a 30 mM phosphate buffer (pH 7.0) to obtain a cell suspension (dry cell: 15 wt.%). Then, a 2.5% aqueous solution of potassium persulfate was mixed with a mixture of acrylamide, N,N'-methylenebisacrylamide, a 5% aqueous solution of N,N,N',N'-tetramethylethylenediamine, the cell suspension, and a 30 mM phosphate buffer to yield a polymer. The final composition is a dry cell concentration 3% ,30 mM phosphate buffer (pH=7) 52%, acrylamide 18%, methylenebisacrylamide 1%, 5% aqueous solution of N,N,N',N'-tetramethylethylenediamine 12%, and 2.5% aqueous solution of potassium persulfate 14% (each % means wt.%). The resulting polymer was cut into particles of about 1 x3x3 mm square to obtain an immobilized cell. The immobilized cell was washed with a 30 mM phosphate buffer (pH=7) to prepare an immobilized cell catalyst (which will hereinafter be called "biocatalyst").

(Hydrolysis using a biocatalyst)

An Erlenmeyer flask having an internal volume of 500 ml was charged with 400 g of distilled water. After a metal mesh basket having therein 1 g (corresponding to 0.03 g of the dry cell) of the biocatalyst obtained above was set in the distilled water and the flask was hermetically sealed with a rubber stopper, the flask was dipped in a temperature controlled water bath to keep the internal temperature at 20°C, followed by stirring with a stirrer.

Acrylonitrile in an amount corresponding to 2 wt.% was fed intermittently (the acrylonitrile concentration was controlled at 0.5 wt.% or greater) and an accumulation reaction of ammonium acrylate was performed. As a result, up to 30 wt.% of ammonium acrylate was accumulated.

The aqueous solution of ammonium acrylate thus obtained was colorless and transparent. 5-L of a reaction mixture was prepared, followed by a purification operation using a UF membrane ("Pencil-type module SIP-0013", product of Asahi Kasei) in the same way. The whole solution was treated without showing a phenomenon such as clogging and a 30 wt. % aqueous solution of ammonium acrylate having a high purity was obtained. As a result, a 30 wt.% aqueous solution of ammonium acrylate having a high purity was obtained. To the resulting aqueous solution was added 200 ppm of methoxyquinone. It was provided for polymerization after concentration to 70 wt.% under light-shielding and pressure-reduced conditions.

To 185.00 g of an aqueous solution of ammonium acrylate thus prepared having a neutralization ratio of 100 mol% was added 0.0021 g of N,N'-methylenebisacrylamide dissolved in 0.5 g of water. The resulting mixture was stirred and dissolved. To the resulting solution was added 0.0920 g of ammonium persulfate dissolved in 0.5 g of water in the same way.

A 2-L separable flask purged with nitrogen in advance and equipped with a reflux condenser was charged with 450 g of cyclohexane and 1.1125 g of sorbitan monostearate as a surfactant. After stirring and dissolving the resulting mixture at room temperature, the aqueous solution of ammonium acrylate obtained above was added. While feeding nitrogen, the resulting mixture was stirred sufficiently at 250 rpm and suspended. Then, polymerization was initiated while reducing the pressure inside the reactor to 65 kPa and keeping the internal temperature at 60°C with a water bath of 60°C.

An emulsion containing a hydrous gel was obtained by retaining the reaction mixture for 2 hours while maintaining the stirring rate at 250 rpm.

The pressure was returned to normal while blowing nitrogen into the reactor and the temperature was raised to 75°C. The stirring rate was set at 300 rpm, then a mixture of 10.4 g of special-grade ethanol produced by Wako Pure Chemicals as an alcohol having a water solubility of 1 wt.% or greater and 0.95 g of water was added over 5 minutes. After large particles were formed by agglomeration, the heating condition was maintained while stirring. Heating was continued for 15 minutes. Then, the solvent was substituted with 450 g of normal-octane having 1.1125 g of sorbitan monostearate dissolved therein. Heating was performed at 100°C for 1 hour to increase the bonding strength.

The hydrous gel thus obtained was collected by filtration, vacuum dried at 100°C and then collected. The primary particle agglomerates thus obtained had an average particle size of 1200 $\mu$m and 6 wt.% of them had a particle size less than 300 $\mu$m. The water absorption ratio as measured by the tea bag method was 75.8 times.

(Manufacturing Example A4)

After 95.04 g of acrylic acid obtained by distilling and purifying special-grade acrylic acid produced by Wako Pure Chemicals was weighed in a 300-ml flask, 199.5 g of 19.9 wt.% aqueous NaOH was added dropwise under stirring while cooling to yield 294.53 g of an aqueous solution of sodium acrylate having a neutralization ratio of 75 mol%. To the resulting aqueous solution was added 0.0305 g of N,N'-methylenebisacrylamide dissolved in 0.5 g of water. The resulting mixture was stirred and dissolved. In addition, 0.0920 g of ammonium persulfate dissolved in 0.5 g of water was added in the same way.

A 2-L separable flask purged with nitrogen in advance and equipped with a reflux condenser was charged with 450 g of cyclohexane and 1.1125 g of sorbitan monostearate as a surfactant. After stirring at room temperature to dissolve them, the aqueous solution of sodium acrylate obtained above was added to the resulting solution. While feeding nitrogen, stirring was performed sufficiently at 200 rpm to obtain a suspension. Then, polymerization was initiated while reducing the pressure inside the reactor to 65 kPa and keeping the internal temperature at 60°C with a water bath of 60°C. The reaction mixture was retained for 2 hours while keeping the stirring rate at 200rpm and an emulsion containing a hydrous gel was obtained.

The pressure was returned to normal while blowing nitrogen into the reactor and the temperature was raised to 75°C. A stirring rate was set at 300 rpm, 8.5 g of special-grade ethanol produced by Wako Pure Chemicals was added as an alcohol having a water solubility of 1 wt.% or greater over 5 minutes. After large particles were formed by agglomeration, the heating condition was maintained while stirring. Heating was continued for one hour. Then, a bath temperature was set at 83°C, a water content of the gel was decreased to 50 wt.% by azeotropic distillation with cyclohexane to reduce the adhesion between gel particles, and the gel was collected.

The hydrous gel thus obtained was collected by filtration, vacuum dried at 100°C and then collected. The primary particle agglomerates thus obtained had an average particle size of 1200 $\mu$m and 6 wt.% of them had a particle size of 300 $\mu$m. They had a water absorption ratio of 65.8 times as measured by the tea bag method and had a resin hardness of 33.7N.

(Manufacturing Example A5)

After 650 g of special-grade acrylic acid produced by Wako Pure Chemicals was weighed in a 2-L flask, 556.1 g of 27.6 wt.% of aqueous ammonia was added dropwise thereto under stirring while cooling to yield 1206.1 g of an aqueous solution of ammonium acrylate having a neutralization ratio of 100 mol%. To the resulting aqueous solution was added 0.0144 g of N,N'-methylenebisacrylamide dissolved in water. The resulting mixture was stirred and dissolved. In addition, 0.6292 g of ammonium persulfate dissolved in water was added in the same way.

A 12-L autoclave purged with nitrogen in advance and equipped with a reflux condenser was charged with 3078 g of cyclohexane and 7.6086 g of sorbitan monostearate as a surfactant. After stirring at room temperature to dissolve them, the aqueous solution of ammonium acrylate obtained above was added to the resulting solution. While feeding nitrogen, stirring was performed sufficiently at 400 rpm to obtain a suspension. While the pressure inside the reactor was reduced and the internal temperature was kept at 70°C by adjusting the jacket temperature at 73°C, polymerization was started. The reaction mixture was retained for 2 hours while keeping the stirring rate at 400 rpm. As a result, an emulsion containing a hydrous gel was obtained.

The pressure was returned to normal while blowing nitrogen into the reactor and the temperature was raised to 75°C. The stirring rate was set at 300 rpm, 58.13 g of special-grade ethanol produced by Wako Pure Chemicals was added as an alcohol having a water solubility of 1 wt.% or greater over 10 minutes. After large particles were formed by agglomeration, the temperature inside of the reactor was heated and pressurized while stirring and the temperature inside the reactor was raised to 110 °C. The temperature was maintained at 110°C while stirring and heating was performed for one hour.

The hydrous gel thus obtained was collected by filtration, vacuum dried at 100°C and then collected. The primary particle agglomerates thus obtained had an average particle of 1200 $\mu$m and 6 wt.% of them had a particle size less than 300 $\mu$m. The water absorption ratio as measured by the tea bag method was 80.2 times and the resin hardness was 13.6N.

(Manufacturing Example A6)

In the same way as Manufacturing Example A2, polymerization was performed. After an emulsion containing a hydrous gel was obtained, the pressure was returned to normal while blowing nitrogen into the inside of the reactor. The temperature was raised to 75°C. A stirring rate was set at 300 rpm, then 8.5 g of special-grade ethanol produced by Wako Pure Chemicals was added as an alcohol having a water solubility of 1 wt.% or greater over 5 minutes. After large particles were formed by agglomeration, the gel was collected without heating.

The hydrous gel thus obtained was collected by filtration, vacuum dried at 100°C and then collected. The average particle size was 1200 $\mu$m and 6 wt.% of them had a particle size less than 300 $\mu$m. The water absorption ratio as measured by the tea bag method was 64.4 times. The resin hardness was very low and unmeasurable.

(Manufacturing Example A7)

95.04 g of acrylic acid obtained by distilling and purifying special-grade acrylic acid produced by Wako Pure Chemicals was weighed in a 300-ml flask. While stirring and cooling, 89.96 g of 25 wt.% aqueous ammonia was added dropwise to yield 185.00 g of an aqueous solution of ammonium acrylate having a neutralization ratio of 100 mol%. To the resulting aqueous solution, 0.0021 g of N,N-methylenebisacrylamide dissolved in 0.5 g of water. The resulting mixture was dissolved by stirring. In addition, 0.092 g of ammonium persulfate dissolved in 0.5 g.of water was added in the same way.

A 2-L separable flask purged with nitrogen in advance and equipped with a reflux condenser was charged with 450 g of cyclohexane and 1.1125 g of sorbitan monostearate as a surfactant. After stirring at room temperature to dissolve them, the aqueous solution of ammonium acrylate obtained above was added to the resulting solution. While feeding nitrogen, stirring was performed sufficiently at 250 rpm to obtain a suspension. Then, polymerization was initiated while reducing the pressure inside the reactor to 65 kPa and keeping the internal temperature at 60°C with a water bath of 60°C. The reaction mixture was retained for 2 hours while keeping a stirring rate at 250 rpm, and an emulsion containing a hydrous gel was obtained.

The pressure was returned to normal while blowing nitrogen into the reactor and the temperature was raised to 75°C. The stirring rate was set at 300 rpm, then a mixture of 8.50 g of special-grade ethanol produced by Wako Pure Chemicals and 1.06 g of special-grade glycerin produced by Wako Pure Chemicals was added as an alcohol having a water solubility of 1 wt.% or greater over 5 minutes. After stirring for 30 minutes, 6 g of special-grade ethanol produced by Wako Pure Chemicals was added further. Stirring was continued. After large particles were formed by agglomeration, the heating condition was maintained while stirring. Heating was continued for one hour.

The hydrous gel thus obtained was collected by filtration, vacuum dried at 100°C and then collected. The primary particle agglomerates thus obtained had an average particle size of 1200 $\mu$m and 6 wt.% of them had a particle size less than 300 $\mu$m. The water absorption ratio as measured by the tea bag method was 75.8 times and the water soluble component amount was 31 %.

(Manufacturing Example A8)

The water absorbing resin produced in Manufacturing Example A7 was heat treated at 180°C for 10 minutes in an inert oven, resulting in a water soluble component amount of 16%.

(Manufacturing Example A9)

The water absorbing resin produced in Manufacturing Example A7 was heat treated at 170°C for 30 minutes in an inert oven, resulting in a water soluble component amount of 8%.

(Manufacturing Example A10)

After 95.04 g of acrylic acid obtained by distilling and purifying special-grade acrylic acid produced by Wako Pure Chemicals was weighed in a 300-ml flask, 89.96 g of 25 wt.% aqueous ammonia was added dropwise while stirring and cooling to obtain 185.00 g of an aqueous solution of ammonium acrylate having a neutralization ratio of 100 mol%. To the resulting aqueous solution, 0.0021 g of N,N'-methylenebisacrylamide dissolved in 0.5 g of water. The resulting mixture was stirred and dissolved. In addition, 0.0920 g of ammonium persulfate dissolved in 0.5 g of water was added in the same way.

In a 2-L separable flask purged with nitrogen in advance and equipped with a reflux condenser was charged with 450 g of cyclohexane and 1.1125 g of sorbitan monostearate as a surfactant. After stirring at room temperature to dissolve them, the aqueous solution of ammonium acrylate obtained above was added to the resulting solution. While feeding nitrogen, stirring was performed sufficiently at 250 rpm to obtain a suspension. Then, polymerization was initiated while reducing the pressure inside the reactor to 65 kPa and keeping the internal temperature at 60°C on a water bath of 60°C. The reaction mixture was retained for 2 hours while keeping the stirring rate at 250 rpm and an emulsion containing a hydrous gel was obtained.

The hydrous gel thus obtained was collected by filtration, vacuum dried at 100°C, and then collected. The primary particle agglomerates thus obtained had an average particle size of 161 $\mu$m and 88.2 wt.% of them had a particle size less than 300 $\mu$m. The water absorption ratio as measured by the tea bag method was 55.8 times.

(Manufacturing Example A11)

In a 100-ml flask, 36 g of purified acrylic acid obtained by distilling special-grade acrylic acid produced by Wako Pure Chemicals and removing a polymerization inhibitor therefrom was weighed. 23.5 g of 36 wt.% aqueous ammonia was added dropwise to obtain 59.5 g of an aqueous solution of ammonium acrylate having a neutralization ratio of 100 mol% while stirring and cooling. To the resulting aqueous solution, 0.0368 g of ammonium persulfate dissolved in 0.5 g of water. The resulting mixture was stirred and dissolved.

In a 500-mL separable flask purged with nitrogen in advance and equipped with a reflux condenser was charged with 180 g of cyclohexane and 0.36 g of sorbitan tristearate as a surfactant. After stirring at room temperature to dissolve them, the aqueous solution of ammonium acrylate obtained above was added to the resulting solution. While feeding nitrogen, stirring was performed sufficiently at 250 rpm to obtain a suspension. Polymerization was then started on a water bath of 55°C, but a stable emulsion was not formed because due to coalescence of the aqueous phase portions, bulk polymerization occurred immediately after polymerization was started.

Manufacturing conditions and physical properties of the water absorbing resin particle agglomerates in Manufacturing Examples A1 to A7, 10, and 11 are shown in Table 2.

[Table 2]

| | | | Manufacturing Example A1 | Manufacturing Example A2 | Manufacturing Example A3 | Manufacturing Example A4 | Manufacturing Example A5 | Manufacturing Example A6 | Manufacturing Example A7 | Manufacturing Example A10 | Manufacturing Example A11 |
|---|---|---|---|---|---|---|---|---|---|---|---|
| Manufacturing conditions of particle agglomerates | Polymerization step | HLB of nonionic surfactant | 4.7 | 4.7 | 4.7 | 4.7 | 4.7 | 4.7 | 4.7 | 4.7 | 2.1 |
| | | Neutralized salt | $NH_3$ | $NH_3$ | $NH_3$ | Na | $NH_3$ | $NH_3$ | $NH_3$ | $NH_3$ | $NH_3$ |
| | | Monomer concentration (wt%) | 65.4 | 63.5 | 63.5 | 40.0 | 66.62 | 63.5 | 63.5 | 63.5 | 74.79 |
| | Agglomeration step | Water soluble solvent | IPA | EtOH | EtOH | EtOH | EtOH | EtOH | EtOH + Glycerol | - | - |
| | Fusion bonding step | Temperature (°C) | 75 | 75 | 100 | 75 | 110 | - | 75 | - | - |
| | | Solvent | Cylo-hexane | Cyclo-hexane | Normal-octane | Cyclo-hexane | Cyclo-hexane | - | Cyclo-hexane | - | - |
| Physical properties of particle agglomerates | Particle size | | 1200 | 1200 | 1200 | 1200 | 1200 | 1200 | 1200 | 161 | - |
| | Water absorption ratio (g/g) | | 70.1 | 65.5 | 75.8 | 65.8 | 80.2 | 64.4 | 75.8 | 55.8 | - |
| | Binding strength (N) | | 7 | 6.5 | 10.1 | 33.7 | 13.6 | Unmeasurable | | - | - |

It has been found from Table 2 that compared with the primary particles obtained in Manufacturing Example A10 which did not correspond to the first embodiment of the present invention, the water absorbing resin agglomerates obtained in Manufacturing Examples A1 to A7 corresponding to the first embodiment of the present invention have an improved absorption ratio and achieved an absorption ratio of 60 g/g or greater which was not achieved by the conventional water absorbing resins.

(Manufacturing Examples B1 to B13)

Manufacturing Examples B1 to B13 of water absorbing resin particle agglomerates/water absorbing resin particles will next be described. Detailed Manufacturing conditions and physical properties of the water absorbing resin particle agglomerates/water absorbing resin particles thus obtained are shown in Table 3.

(Manufacturing Example B1)

In a 500-ml flask, 211.8 g of acrylic acid obtained by distilling and purifying special-grade acrylic acid produced by Wako Pure Chemicals was weighed. 188.5 g of 26.5 wt.% aqueous ammonia was added dropwise while stirring and cooling to obtain 400.3 g of an aqueous solution of ammonium acrylate having a neutralization ratio of 100 mol%.
To the resulting solution was added 0.026 g of N,N'-methylenebisacrylamide dissolved in 0.5 g of water as a radical polymerizable crosslinking agent. The resulting mixture was stirred and dissolved. 0.1081 g of ammonium persulfate dissolved in 0.5 g of water was added as a polymerization initiator in the same way.
In a 2-L separable flask purged with nitrogen in advance and equipped with a reflux condenser was charged with 400.0 g of cyclohexane and 1.9 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 200 rpm to obtain a suspension. Then, polymerization was initiated while reducing the pressure inside the reactor to 65 kPa and keeping the internal temperature at 60°C with a water bath of 60°C. The reaction mixture was retained for 2 hours while keeping the stirring rate at 200 rpm and an emulsion containing a hydrous gel was obtained.
The pressure was returned to normal while blowing nitrogen into the reactor and the temperature was raised with a hot water bath of 75°C. The stirring rate was set at 300 rpm, then 11.0 g of isopropanol produced by Wako Pure Chemicals and 3.46 g of special-grade glycerin produced by Wako Pure Chemicals were added as a water soluble solvent having a water solubility of 1 wt.% or greater over 5 minutes. Stirring was continued for 30 minutes. Then, 6.5 g of isopropanol was added further. After large particles were formed by agglomeration, the heating state was maintained while stirring. Heating was continued for one hour.
The hydrous gel thus obtained was collected by filtration, vacuum dried at 100°C and collected.
The water absorbing resin particle agglomerates thus formed were heat treated at 180°C for 15 minutes in an inert oven. The water absorbing resin particle agglomerates thus obtained had an average particle size of 1200 μm and the primary particles had a particle size of 161 μm.

The water absorbing resin particle agglomerates thus obtained were sifted using sieves having openings of 850 μm and 1400 μm, respectively to remove the particles which had remained on the sieve of 1400 μm and the particles which had passed through the sieve of 850 μm. The neutralization ratio on the outer surface, the neutralization ratio inside, the initial water absorption rate, water absorption ratio, water soluble component amount, and bonding strength of the agglomerates were measured.

(Manufacturing Example B2)

In a 300-ml flask, 95.0 g of acrylic acid obtained by distilling and purifying special-grade acrylic acid produced by Wako Pure Chemicals was weighed. 90.0 g of 25 wt.% aqueous ammonia was added dropwise to obtain 185.0 g of an aqueous solution of ammonium acrylate having a neutralization ratio of 100 mol% while stirring and cooling.
To the resulting solution was added 0.0027 g of N,N'-methylenebisacrylamide dissolved in 0.5 g of water. The resulting mixture was stirred and dissolved. To the resulting solution was added 0.0920 g of ammonium persulfate dissolved in 0.5 g of water in the same way.
In a 2-L separable flask purged with nitrogen in advance and equipped with a reflux condenser was charged with 450.0 g of cyclohexane and 1.1 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 200 rpm to obtain a suspension. Then, polymerization was initiated while reducing the pressure inside the reactor to 65 kPa and keeping the internal temperature at 60°C with a water bath of 60°C. The reaction mixture was retained for 2 hours while keeping the stirring rate kept at 200 rpm to obtain an emulsion containing a hydrous gel.
The pressure was returned to normal while blowing nitrogen into the reactor and the temperature was raised with a hot water bath of 75°C. The stirring rate was set at 300 rpm, then 8.5 g of special-grade ethanol produced by Wako Pure Chemicals was added over 5 minutes as a water soluble solvent having a water solubility of 1 wt.% or greater. Stirring was continued for 30 minutes. Then, 6.0 g of special-grade ethanol produced by Wako Pure Chemicals was added further and stirring was continued. After large particles were formed by agglomeration, the heating condition was maintained while stirring. Heating was continued for one hour.
The hydrous gel thus formed was collected by filtration, followed by vacuum drying at 100°C.
The water absorbing resin particle agglomerates thus formed were heat treated at 180°C for 10 minutes in an inert oven.
The water absorbing resin particle agglomerates thus obtained had an average particle size of 1200 μm and its primary particles had a particle size of 120 μm.
The water absorbing resin particle agglomerates thus obtained were sifted using sieves having openings of 850 μm and 1400 μm, respectively to remove the particles which had remained on the sieve of 1400 μm and the particles which had passed through the sieve of 850 μm. The neutralization ratio on the outer surface, neutralization ratio inside, initial water absorption rate, water absorption ratio, water soluble component amount, and bonding strength of the agglomerates were measured.
(Manufacturing Example B3)
Ammonium acrylate was prepared in the following manner.

(Preparation of biocatalyst)

*Acinetobacter sp.* AK226 (FERM BP-08590) having a nitrilase activity was aerobically cultured at 30°C on a culture medium adjusted to pH 7 with an aqueous solution containing 0.1 % of sodium chloride, 0.1% of potassium dihydrogen phosphate, 0.05 % of magnesium sulfate heptahydrate, 0.005% of iron sulfate heptahydrate, 0.005% of managanese sulfate pentahydrate, 0.1% of ammonium sulfate, and 0.1% of potassium nitrate (each, weight%) by adding 0.5 wt.% of acetonitrile as a nutrition source to the culture medium. The resulting culture medium was washed with a 30 mM phosphate buffer (pH 7.0) to obtain a cell suspension (dry cell: 15 wt.%). Then, a 2.5% aqueous solution of potassium persulfate was mixed with a mixture of acrylamide, N,N'-methylenebisacrylamide, a 5% aqueous solution of N,N,N',N'-tetramethylethylenediamine, the cell suspension, and a 30 mM phosphate buffer to yield a polymer. The final composition is a dry cell concentration 3% ,a 30 mM phosphate buffer (pH=7) 52%, acrylamide 18%, methylenebisacrylamide 1% , a 5% aqueous solution of N,N,N',N'-tetramethylethylenediamine 12%, and a 2.5% aqueous solution of potassium persulfate 14% (each % means wt.%). The resulting polymer was cut into particles of about 1×3×3 mm square to obtain an immobilized cell. The immobilized cell was washed with a 30 mM phosphate buffer (pH=7) to prepare an immobilized cell catalyst (which will hereinafter be called "biocatalyst").

(Hydrolysis using a biocatalyst)

An Erlenmeyer flask having an internal volume of 500 ml was charged with 400 g of distilled water. After a metal mesh basket having therein 1 g (corresponding to 0.03 g of the dry cell) of the biocatalyst obtained above was set in the distilled water and the flask was hermetically sealed with a rubber stopper, the flask was dipped in a temperature controlled water bath to keep the internal temperature at 20°C, followed by stirring with a stirrer.

Acrylonitrile in an amount corresponding to 2 wt.% was fed intermittently (the acrylonitrile concentration was controlled at 0.5 wt.% or greater) and an accumulation reaction of ammonium acrylate was performed. As a result, up to 30 wt.% of ammonium acrylate was accumulated.

The aqueous solution of ammonium acrylate thus obtained was colorless and transparent. 5L of a reaction mixture was prepared in the same way, followed by a purification operation using a UF membrane ("Pencil-type module SIP-0013", product of Asahi Kasei). The whole solution was treated without showing a phenomenon such as clogging and a 30 wt. % aqueous solution of ammonium acrylate having a high purity was obtained. To the resulting aqueous solution was added 200 ppm of methoxyquinone and the resulting mixture was concentrated to 70 wt.% under light-shielding and pressure-reduced conditions.

The aqueous solution (185.0 g) of ammonium acrylate thus prepared having a neutralization ratio of 100 mol% was used.

To the resulting aqueous solution was added 0.0021 g of N,N'-methylenebisacrylamide dissolved in 0.5 g of water. The resulting mixture was stirred and dissolved. In addition, 0.0920 g of ammonium persulfate dissolved in 0.5 g of water was added in the same way.

In a 2-L separable flask purged with nitrogen in advance and equipped with a reflux condenser was charged with 450.0 g of cyclohexane and 1.1 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 250 rpm to obtain a suspension. Then, polymerization was initiated while reducing the pressure inside the reactor to 65 kPa and keeping the internal temperature at 60°C with a water bath of 60°C. The reaction mixture was retained for 2 hours while keeping the stirring ratet at 250 rpm, to obtain an emulsion containing a hydrous gel.

The pressure was returned to normal while blowing nitrogen into the reactor and the temperature was raised with a hot bath of 75°C. The stirring rate was set at 300 rpm, then a mixture of 10.4 g of special-grade ethanol produced by Wako Pure Chemicals which was a water soluble solvent having a water solubility of 1 wt.% or greater and 1.0 g of water was added over 5 minutes. After large particles were formed by agglomeration, a heated state was kept while stirring, followed by heating for 15 minutes. Then, the solvent was substituted with 450 g of normal-octane having 1.1125 g of sorbitan monostearate dissolved therein. The solution was heated at 100°C for 1 hour to increase the bonding strength of the particles.

The hydrous gel thus obtained was collected by filtration, vacuum dried at 100°C and then collected.

The water absorbing resin particle agglomerates thus formed were heat treated at 180°C for 10 minutes in an inert oven. The water absorbing resin particle agglomerates thus obtained had an average particle size of 1350 $\mu$m and their primary particles had a particle size of 120 $\mu$m.

The water absorbing resin particle agglomerates thus obtained were sifted using sieves having openings of 850 $\mu$m and 1400 $\mu$m, respectively to remove the particles which had remained on the sieve of 1400 $\mu$m and the particles which had passed through the sieve of 850 $\mu$m. The neutralization ratio on the outer surface, neutralization ratio inside, water absorption ratio, and bonding strength of the agglomerates were measured.

(Manufacturing Example B4)

Special-grade acrylic acid (650 g) produced by Wako Pure Chemicals was weighed in a 2-L flask. While stirring and cooling, 556 g of 27.6 wt.% aqueous ammonia was added dropwise to yield 1206 g of an aqueous solution of ammonium acrylate having a neutralization ratio of 100 mol%.

To the resulting aqueous solution was added 0.0144 g of N,N'-methylenebisacrylamide dissolved in water. The resulting mixture was stirred and dissolved. In addition, 0.6292 g of ammonium persulfate dissolved in water was added in the same way.

A 12-L autoclave purged with nitrogen in advance and equipped with a reflux condenser was charged with 3078 g of cyclohexane and 8 g of sorbitan monostearate as a surfactant. After stirring at room temperature to dissolve them, the aqueous solution of ammonium acrylate obtained above was added to the resulting solution. While feeding nitrogen, stirring was performed sufficiently at 400 rpm to obtain a suspension. Then, polymerization was initiated while leaving the inside of the reactor in a pressure-reduced state, raising the temperature to a jacket temperature of 73°C, and keeping the internal temperature at 70°C. An emulsion containing a hydrous gel was obtained by retaining the reaction mixture for 2 hours while maintaining the stirring rate at 100 rpm.

The pressure was returned to normal while blowing nitrogen into the reactor and the temperature was raised by a jacket temperature of 75°C. The stirring rate was set at 300 rpm, then a mixture of 55 g of special-grade ethanol produced by Wako Pure Chemicals as an alcohol having a water solubility of 1 wt.% in water and 7 g of special-grade glycerin produced by Wako Pure Chemicals was added over 5 minutes. After stirring for 30 minutes, 20 g of special-grade ethanol produced by Wako Pure Chemicals was added and stirring was continued. After large particles were formed by agglomeration, the temperature inside of the reactor was heated and pressurized while stirring and the temperature inside the reactor was raised to 110 °C. The temperature inside the reactor was maintained at 110°C while stirring and heating was performed for one hour.

The hydrous gel thus obtained was collected by filtration, vacuum dried at 100°C, and then collected.

The water absorbing resin agglomerates thus formed were heated at 180°C for 10 minutes in an inert oven. The water absorbing resin particle agglomerates thus obtained had an average particle size of 1420 $\mu$m and their primary particles had a particle size of 100 $\mu$m.

The water absorbing resin particle agglomerates thus obtained were sifted using sieves having openings of 850 $\mu$m and 1400 $\mu$m, respectively to remove the particles which had remained on the sieve of 1400 $\mu$m and the particles which had passed through the sieve of 850 $\mu$m. The neutralization ratio on the outer surface, neutralization ratio inside, initial water absorption rate, water absorption ratio, bonding strength, and water soluble component amount of the agglomerates were measured.

(Manufacturing Example B5)

In a 300-ml flask, 95.0 g of acrylic acid obtained by distilling and purifying special-grade acrylic acid produced by Wako Pure Chemicals was weighed. While stirring and cooling, 90.0 g of 25 wt.% aqueous ammonia was added dropwise to obtain 185.0 g of an aqueous solution of ammonium acrylate having a neutralization ratio of 100 mol%.

To the resulting solution was added 0.0027 g of N,N'-methylenebisacrylamide dissolved in 0.5 g of water. The resulting mixture was stirred and dissolved. To the resulting solution was added 0.0920 g of ammonium persulfate dissolved in 0.5 g of water in the same way.

In a 2-L separable flask purged with nitrogen in advance and equipped with a reflux condenser was charged with 450.0 g of cyclohexane and 1.1 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 200 rpm to obtain a suspension. Then, polymerization was initiated while reducing the pressure inside the reactor to 65 kPa and keeping the internal temperature at 60°C with a water bath of 60°C. The reaction mixture was retained for 2 hours while keeping the stirring rate at 200rpm to obtain an emulsion containing a hydrous gel.

The pressure was returned to normal while blowing nitrogen into the reactor and the temperature was raised to 75°C. At a stirring rate set at 300 rpm, 8.5 g of special-grade ethanol produced by Wako Pure Chemicals was added over 5 minutes as an alcohol having a water solubility of 1 wt.% or greater. After large particles were formed by agglomeration, the resulting gel was collected without heating.

The hydrous gel thus formed was collected by filtration, vacuum-dried at 100°C, and collected.

The water absorbing resin particle agglomerates thus formed were heat treated at 170°C for 30 minutes in an inert oven. The water absorbing resin particle agglomerates thus obtained had an average particle size of 1200 $\mu$m and their primary particle size had a diameter of 120 $\mu$m.

The water absorbing resin particle agglomerates thus obtained were sifted through sieves having openings of 850 $\mu$m and 1400 $\mu$m, respectively to remove the particles which had remained on the sieve of 1400 $\mu$m and the particles which had passed through the sieve of 850 $\mu$m. The neutralization ratio on the outer surface, neutralization ratio inside, initial water absorption rate, water absorption ratio, and bonding strength of the agglomerates were measured.

(Manufacturing Example B6)

In a 300-ml flask, 95.04 g of acrylic acid obtained by distilling and purifying special-grade acrylic acid produced by Wako Pure Chemicals was weighed. While stirring and cooling, 89.96 g of 25 wt.% aqueous ammonia was added dropwise to obtain 185.00 g of an aqueous solution of ammonium acrylate having a neutralization ratio of 100 mol%.

To the resulting solution was added 0.0021 g of N,N'-methylenebisacrylamide dissolved in 0.5 g of water. The resulting mixture was stirred and dissolved. To the resulting solution was added 0.0920 g of ammonium persulfate dissolved in 0.5 g of water in the same way.

In a 2-L separable flask purged with nitrogen in advance and equipped with a reflux condenser was charged with 450 g of cyclohexane and 1.1125 g of sorbitan monostearate as a surfactant. After stirring at room temperature to dissolve them, the aqueous solution of ammonium acrylate obtained described above was added to the resulting solution. While feeding nitrogen, the mixture was stirred sufficiently at 250 rpm to obtain a suspension. Then, polymerization was initiated while reducing the pressure inside the reactor to 65 kPa and keeping the internal temperature at 60°C on a water bath of 60°C. At a stirring rate kept at 250 rpm, the reaction mixture was retained for 2 hours and an emulsion containing a hydrous gel was obtained.

The pressure was returned to normal while blowing nitrogen into the reactor and the temperature was raised to 75°C. At a stirring rate set at 300 rpm, a mixture of 8.50 g of special-grade ethanol produced by Wako Pure Chemicals and 1.06 g of special-grade glycerin produced by Wako Pure Chemicals was added as an alcohol having a water solubility of 1 wt.% or greater. Stirring was continued for 30 minutes. Then, 6 g of special-grade ethanol produced by Wako Pure Chemicals was added further and stirring was continued. After large particles were formed by agglomeration, the heating state was maintained while stirring. Heating was continued for three hours.

The hydrous gel thus formed was collected by filtration, vacuum-dried at 100°C. and collected.

The water absorbing resin particle agglomerates thus formed were heat treated at 180°C for 10 minutes in an inert oven. The water absorbing resin particle agglomerates thus obtained had an average particle size of 1200 $\mu$m and their primary particles had a particle size of 120 $\mu$m.

The water absorbing resin particle agglomerates thus obtained were sifted through sieves having openings of 850 $\mu$m and 1400 $\mu$m, respectively to remove the particles which had remained on the sieve of 1400 $\mu$m and the particles which had passed through the sieve of 850 $\mu$m. The neutralization ratio on the outer surface, neutralization ratio inside, water absorption ratio, and bonding strength of the agglomerates were measured.

(Manufacturing Example B7)

Acrylic acid (753 g) obtained by distilling and purifying special-grade acrylic acid produced by Wako Pure Chemicals was weighed. While stirring and cooling to a liquid temperature not greater than 30°C by ice cooling, 625 g of 25 wt.% special-grade aqueous ammonia produced by Wako Pure Chemicals was added dropwise to obtain 1378 g of an aqueous solution of ammonium acrylate having a neutralization ratio of 100 mol%.

To the resulting aqueous solution was added 0.7699 g of ammonium persulfate dissolved in 50 g of water.

A 12-L autoclave purged with nitrogen and equipped with a reflux condenser was charged with 3350 g of cyclohexane and 7.53 g of sorbitan monolaurate as a surfactant. The resulting mixture was stirred and dissolved. Then, the aqueous solution of ammonium acrylate obtained described above was added to the resulting solution. While feeding nitrogen, the mixture was stirred sufficiently at 400 rpm to obtain a suspension. Polymerization was then started while reducing the pressure inside the reactor to 30 kPa and keeping the internal temperature at 40°C with a water bath of 60°C. The reaction mixture was retained for 2 hours while keeping the stirring rate at 400rpm to obtain an emulsion containing a hydrous gel was obtained.

The pressure was returned to normal while blowing nitrogen into the reactor and the temperature was raised to 75°C. The stirring rate was set at 500 rpm, then a mixture of 35 g of special-grade isopropanol produced by Wako Pure Chemicals and 8 g of special-grade glycerin produced by Wako Pure Chemicals was added over 5 minutes as an alcohol having a water solubility of 1 wt.% or greater. Stirring was continued for 30 minutes. Then, 25 g of special-grade isopropanol produced by Wako Pure Chemicals was added further and stirring was continued. After large particles were formed by agglomeration, the heating condition was maintained while stirring. Heating was continued for three hours.

The hydrous gel thus formed was collected by filtration, vacuum dried at 100°C, and collected.

The water absorbing resin particle agglomerates thus formed were heat treated at 170°C for 30 minutes in an inert oven. The water absorbing resin particle agglomerates thus obtained had an average particle size of 3000 $\mu$m and their primary particles had a particle size of 700 $\mu$m.

The water absorbing resin particle agglomerates thus obtained were sifted using sieves having openings of 850 $\mu$m and 1400 $\mu$m, respectively to remove the particles which had remained on the sieve of 1400 $\mu$m and the particles which had passed through the sieve of 850 $\mu$m. The neutralization ratio on the outer surface, neutralization ratio inside, initial water absorption rate, water absorption ratio, and bonding strength of the agglomerates were removed were measured.

(Manufacturing Example B8)

After 18 g of acrylic acid obtained by distilling and purifying special-grade acrylic acid produced by Wako Pure Chemicals was weighed, 13 g of water was added thereto. While stirring and cooling to a liquid temperature not greater than 30°C by ice, 18 g of 25 wt.% aqueous ammonia, the special-grade product produced by Wako Pure Chemicals, was added dropwise to obtain 56 g of an aqueous solution of ammonium acrylate having a neutralization ratio of 100 mol%.

To the resulting aqueous solution was added 0.0004 g of N, N'-methylenebisacrylamide dissolved in 0.5 g of water. The resulting mixture was stirred and dissolved. To the resulting solution was added 0.0184 g of ammonium persulfate dissolved in 0.1 g of water.

A 500-cc separable flask purged with nitrogen and equipped with a reflux condenser was charged with 90 g of cyclohexane and 0.18 g of sorbitan monolaurate as a surfactant. The resulting mixture was stirred and dissolved at room temperature. The aqueous solution of ammonium acrylate obtained above was then added to the resulting solution. While feeding nitrogen, the mixture was stirred sufficiently at 400 rpm to obtain a suspension. Polymerization was then started while reducing the pressure inside the reactor to 65 kPa and keeping the internal temperature at 60°C with a water bath of 63°C. The reaction mixture was retained for 2 hours while keeping the stirring rate at 400 rpm and an emulsion containing a hydrous gel was obtained.

The pressure was returned to normal while blowing nitrogen into the reactor and the temperature was raised to 75°C. The stirring rate was set at 500 rpm, then a mixture of 1.6 g of special-grade ethanol produced by Wako Pure Chemicals and 0.2 g of special-grade glycerin produced by Wako Pure Chemicals was added over 5 minutes as an alcohol having a water solubility of 1 wt.% or greater. Stirring was continued for 30 minutes. Then, 1.1 g of special-grade ethanol produced by Wako Pure Chemicals was added further and stirring was continued. After large particles were formed by agglomeration, the heating condition was maintained while stirring. Heating was continued for three hours.

The hydrous gel thus formed was collected by filtration, vacuum dried at 100°C, and collected.

The water absorbing resin particle agglomerates thus formed were heat treated at 170°C for 30 minutes in an inert oven. The water absorbing resin particle agglomerates thus obtained had an average particle size of 900 $\mu$m and their primary particles had a particle size of 120 $\mu$m.

The water absorbing resin particle agglomerates thus obtained was sifted using sieves having openings of 850 $\mu$m and 1400 $\mu$m, respectively to remove the particles which had remained on the sieve of 1400 $\mu$m and the particles which had passed through the sieve of 850 $\mu$m. The neutralization ratio on the outer surface, neutralization ratio inside, water absorption ratio, and bonding strength of the agglomerates were measured.

(Manufacturing Example B9)

In a 300-ml flask, 95.04 g of acrylic acid obtained by distilling and purifying special-grade acrylic acid produced by Wako Pure Chemicals was weighed. While stirring and cooling, 89.96 g of 25 wt.% aqueous ammonia was added dropwise to obtain 185.00 g of an aqueous solution of ammonium acrylate having a neutralization ratio of 100 mol%.

To the resulting solution was added 0.0021 g of N,N'-methylenebisacrylamide dissolved in 0.5 g of water. The resulting mixture was stirred and dissolved. To the resulting solution was added 0.0920 g of ammonium persulfate dissolved in 0.5 g of water in the same way.

In a 2-L separable flask purged with nitrogen in advance and equipped with a reflux condenser was charged with 450 g of cyclohexane and 1.1125 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 250 rpm to obtain a suspension. Polymerization was then started while reducing the pressure inside the reactor to 65 kPa and keeping the internal temperature at 60°C with a water bath of 60°C. The reaction mixture was retained for 2 hours while keeping the stirring rate at 250 rpm to obtain an emulsion containing a hydrous gel.

The hydrous gel thus obtained was collected by filtration, vacuum dried at 100°C, and collected.

The water absorbing resin particles thus obtained were heat treated at 180°C for 10 minutes in an inert oven. The water absorbing resin particles thus obtained had an average particle size of 161 $\mu$m.

The neutralization ratio on the outer surface, neutralization ratio inside, water absorption ratio, and bonding strength of the water absorbing resin particles were measured.

(Manufacturing Example B10)

In a 300-ml flask, 95.0 g of acrylic acid obtained by distilling and purifying special-grade acrylic acid produced by Wako Pure Chemicals was weighed. While stirring and cooling, 90.0 g of 25 wt.% aqueous ammonia was added dropwise to obtain 185.0 g of an aqueous solution of ammonium acrylate having a neutralization ratio of 100 mol%.

To the resulting solution was added 0.0027 g of N,N'-methylenebisacrylamide dissolved in 0.5 g of water. The resulting mixture was stirred and dissolved. To the resulting solution was added 0.0920 g of ammonium persulfate dissolved in 0.5 g of water in the same way.

In a 2-L separable flask purged with nitrogen in advance and equipped with a reflux condenser was charged with 450.0 g of cyclohexane and 1.1 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 200 rpm to obtain a suspension. Polymerization was then started while reducing the pressure inside the reactor to 65 kPa and keeping the internal temperature at 60°C with a water bath of 60°C. The reaction mixture was retained for 2 hours while keeping the stirring rate at 200 rpm to obtain a hydrous gel was obtained.

The pressure was returned to normal while blowing nitrogen into the reactor and the temperature was raised with a hot bath of 75°C. The stirring rate was set at 300 rpm, 8.5 g of special-grade ethanol produced by Wako Pure Chemicals was added over 5 minutes. After stirring for 30 minutes, 6.0 g of special-grade ethanol produced by Wako Pure Chemicals was added and stirring was continued. After large particles were formed by agglomeration, the heating condition was maintained while stirring, and heating was continued for one hour.

The hydrous gel thus formed was collected by filtration, vacuum dried at 100°C, and collected.

The water absorbing resin particle agglomerates thus formed were heat treated at 120°C for 60 minutes in an inert oven. The water absorbing resin particle agglomerates thus obtained had an average particle size of 1200 μm and their primary particles had a particle size of 120 μm.

The water absorbing resin particle agglomerates thus obtained were sifted using sieves having openings of 850 μm and 1400 μm, respectively to remove the particles which had remained on the sieve of 1400 μm and the particles which had passed through the sieve of 850 μm. The neutralization ratio on the outer surface, neutralization ratio inside, water absorption ratio, water soluble component amount, and bonding strength of the agglomerate from which the particles were measured.

The water absorbing resin particle agglomerates caused gel blocking due to a *Mamako* phenomenon during the measurement of a water absorption ratio and they did not absorb water as a whole.

(Manufacturing Example B11)

In a 300-ml flask, 95.0 g of acrylic acid obtained by distilling and purifying special-grade acrylic acid produced by Wako Pure Chemicals was weighed and dissolved in 40.7 g of distilled water. While stirring and cooling, 49.3 g of 25 wt.% aqueous ammonia was added dropwise to obtain 185.0 g of an aqueous solution of ammonium acrylate having a neutralization ratio of 55 mol%.

To the resulting solution was added 0.0035 g of N,N'-methylenebisacrylamide dissolved in 0.5 g of water. The resulting mixture was stirred and dissolved. To the resulting solution was added 0.0934 g of ammonium persulfate dissolved in 0.5 g of water in the same way.

In a 2-L separable flask purged with nitrogen in advance and equipped with a reflux condenser was charged with 450.0 g of cyclohexane and 1.1 g of sorbitan monostearate as a surfactant. After stirring at room temperature to dissolve them, the aqueous solution of ammonium acrylate obtained above was added to the resulting solution. While feeding nitrogen, the mixture was stirred sufficiently at 200 rpm to obtain a suspension. Polymerization was then started while reducing the pressure inside the reactor to 65 kPa and keeping the internal temperature at 60°C with a water bath of 60°C. The reaction mixture was retained for 2 hours while keeping the stirring rate at 200 rpm to obtain an emulsion containing a hydrous gel.

The pressure was returned to normal while blowing nitrogen into the reactor and the temperature was raised with a hot bath of 75°C. The stirring rate was set at 300 rpm, then 8.5 g of special-grade ethanol produced by Wako Pure Chemicals was added over 5 minutes. After stirring for 30 minutes, 6.0 g of special-grade ethanol produced by Wako Pure Chemicals was added and stirring was continued. After large particles were formed by agglomeration, the heating condition was maintained while stirring and heating was continued for one hour.

The water absorbing resin particle agglomerates thus formed were heat treated at 150°C for 30 minutes in an inert oven. The water absorbing resin particle agglomerates thus obtained had an average particle size of 1000 μm and their primary particles had a particle size of 100 μm.

The water absorbing resin particle agglomerates thus obtained were sifted using sieves having openings of 850 μm and 1400 μm, respectively to remove the particles which had remained on the sieve of 1400 μm and the particles which had passed through the sieve of 850 μm. The neutralization ratio on the outer surface, neutralization ratio inside, water absorption ratio, and bonding strength of the agglomerates were measured.

The water absorbing resin particle agglomerates caused gel blocking, so-called "*Mamako* phenomenon", during the measurement of a water absorption ratio.

(Manufacturing Example B12)

In a 100-ml flask, 36 g of acrylic acid purified by distilling special-grade acrylic acid produced by Wako Pure Chemicals and removing a polymerization inhibitor was weighed in a 100-ml flask. While stirring and cooling, 23.5 g of 36 wt.% aqueous ammonia was added dropwise to yield 59.5 g of an aqueous solution of ammonium acrylate having a neutralization ratio of 100 mol%.

To the resulting solution was added 0.0368 g of ammonium persulfate dissolved in 0.5 g of water. The resulting mixture was stirred and dissolved.

A 500-ml separable flask purged with nitrogen in advance and equipped with a reflux condenser was charged with 180 g of cyclohexane and 0.36 g of sorbitan tristearate as a surfactant. After the resulting mixture was stirred and dissolved at room temperature, the aqueous solution of ammonium acrylate obtained above was added. While feeding nitrogen, the mixture was stirred sufficiently at 250 rpm to form a suspension. Then, polymerization was started with a water bath of 55°C. Due to coalescence of the aqueous phase portions immediately after initiation of the polymerization, bulk polymerization occurred and a stable emulsion was not obtained.

(Manufacturing Example B13)

A 40 wt.% aqueous solution of ammonium acrylate having a neutralization ratio of 100 mol% was prepared in the same way as Manufacturing Example B3 except that the aqueous solution was concentrated to 40 wt.% in a 300-ml separable flask.
To the aqueous solution of ammonium acrylate was added 0.0187 g of N, N'-methylenebisacrylamide.
The flask was kept warm with a water bath so as to keep the liquid temperature at 30°C. The deaeration of the aqueous solution was performed by bubbling with a nitrogen gas and the reaction system was purged with nitrogen. To the reaction mixture was added 0.86 g of a 42 wt.% aqueous glycerin solution through a syringe. After stirring thoroughly, 0.0917 g of a 30 wt.% aqueous solution of hydrogen peroxide and 0.0415 g of Longarit, each dissolved in 1 g of water were added and polymerization was initiated. The internal temperature was raised from 30°C to 100°C over 10 minutes. Then, heating was conducted for 3 hours with a water bath so as to keep the internal temperature at 70°C.
The gel thus obtained was then taken out from the separable flask, followed by rough crushing and then drying at 100°C in a vacuum dryer. After completion of the drying, the roughly crushed gel was pulverized in a homogenizer and particles having a particle size of from 850 to 1000 μm were collected by sifting. The water absorbing resin particles thus obtained had an average particle size of 925 μm.
The water absorbing resin particle agglomerates thus obtained were sifted using sieves having openings of 850 μm and 1400 μm, respectively to remove the particles which had remained on the sieve of 1400 μm and the particles which had passed through the sieve of 850 μm. The initial water absorption rate and water absorption ratio of the agglomerates were measured.

Manufacturing conditions of Manufacturing Examples B1 to B13, and physical properties of the water absorbing resin particle agglomerates are shown in Table 3.

## [Table 3]

| | | | Manufacturing Ex. B1 | Manufacturing Ex. B2 | Manufacturing Ex. B3 | Manufacturing Ex. B4 | Manufacturing Ex. B5 | Manufacturing Ex. B6 | Manufacturing Ex. B7 | Manufacturing Ex. B8 | Manufacturing Ex. B9 | Manufacturing Ex. B10 | Manufacturing Ex. B11 | Manufacturing Ex. B12 | Manufacturing Ex. B13 |
|---|---|---|---|---|---|---|---|---|---|---|---|---|---|---|---|
| Manufacturing conditions of particle agglomerate | Polymerization step | HLB of nonionic surfactant | 4.7 | 4.7 | 4.7 | 4.7 | 4.7 | 4.7 | 8.6 | 4.7 | 4.7 | 4.7 | 4.7 | 2.1 | - |
| | | Neutralization ratio of monomer with $NH_3$ (mol%) | 100 | 100 | 100 | 100 | 100 | 100 | 100 | 100 | 100 | 100 | 55 | 100 | 100 |
| | | Monomer concentration (wt.%) | 65.4 | 63.5 | 63.5 | 66.6 | 63.5 | 63.5 | 67.5 | 45.0 | 63.5 | 63.5 | 58.0 | 63.6 | 40.0 |
| | Agglomeration step | Water soluble solvent | IPA+Glycerol | EtOH | EtOH | EtOH+Glycerol | EtOH | EtOH+Glycerol | IPA+Glycerol | EtOH+Glycero | - | EtOH | EtOH | - | - |
| | Fusion bonding step | Temperature (°C) | 75 | 75 | 100 | 110 | - | 75 | 75 | 75 | - | 75 | 75 | - | - |
| | | Solvent | Cyclo-hexane | Cyclo-hexane | Normal octane | Cyclo-hexane | Cyclo-hexane | Cyclo-hexane | Cyclo-hexane | Cyclo-hexane | | Cyclo-hexane | Cyclo-hexane | - | - |
| | Heat treatment step | Temperature (°C) | 180 | 180 | 180 | 180 | 170 | 180 | 170 | 170 | 180 | 120 | 150 | - | - |
| | | Time (minute) | 15 | 10 | 10 | 10 | 30 | 10 | 30 | 30 | 10 | 60 | 30 | - | - |
| Physical properties of particle agglomerate | Carboxyl neutralization ratio (mol%) | Outer surface | 20 | 10 | 10 | 10 | 30 | 10 | 30 | 10 | 8 | 80 | 45 | - | - |
| | | Inside | 80 | 60 | 60 | 60 | 80 | 60 | 90 | 70 | 60 | 60 | 50 | - | - |
| | Particle size | Primary particles | 161 | 120 | 120 | 100 | 120 | 120 | 700 | 120 | 161 | 120 | 100 | - | 925 |
| | | Secondary particles | 1200 | 1200 | 1350 | 1420 | 1200 | 1200 | 3000 | 900 | - | 1200 | 1000 | - | - |
| | Water absorption ratio (g/g) | | 73.1 | 75.3 | 80.4 | 74.2 | 64.4 | 75.3 | 82.3 | 74.7 | 53.4 | 50.1 (Mamako) | 51.3 (Mamako) | - | 55.4 |
| | Initial water absorption rate (g/g) | | 13 | 19 | - | 20 | 19 | - | 9 | - | - | - | - | - | 8 |
| | Bonding strength (N) | | 7 | 6.5 | 10.1 | 14.1 | Unmeasurable | 9.3 | 6.5 | 6.3 | - | 9.8 | 10.1 | - | - |
| | Water soluble component amount (%) | | 10 | - | - | 16 | - | - | - | - | - | 30 | - | - | - |

The water absorbing resin particle agglomerates obtained in Manufacturing Examples 1 to 8 corresponding to the second embodiment of the present invention show a high water absorption ratio and initial water absorption rate. From the comparison with the water absorbing resin particle agglomerates obtained in Manufacturing Examples 10 and 11 which did not correspond to the second embodiment of the present invention, it has been confirmed that the mamako phenomenon of the water absorbing resin particle agglomerates can be prevented and a high water absorption ratio can be achieved by controlling the neutralization ratio on the outer surface and inside of the agglomerates to fall within a range specified in the second embodiment of the present invention.

(Body fluid absorption articles)

Body fluid absorption articles produced using the water absorbing resin particle agglomerates (1) manufactured in Manufacturing Example B1 will hereinafter be described.

"Bemliese" (trade mark) produced by Asahi Kasei Fibers ("Bemliese" is a continuous long-fibered nonwoven fabric made of 100% cotton. Because it is a cellulosic nonwoven fabric, it has excellent water absorption properties. Because it is made of continuous long fibers, it has sufficient strength when it contains water, and has excellent liquid dispersibility. Physical properties of Bemliese are shown in Table 4) cut into a circle having a diameter of 59.5 mm was prepared as a base material. As a result of measurement, the base material had a weight of 0.0796 g.

Two Teflon sheets having a diameter of 59.5 mm were prepared. Of the water absorbing resin particle agglomerates (1) synthesized in Manufacturing Example B1, 0.164 g of the agglomerates having an average particle size of from 850 to 1200 $\mu$m were placed so as not to contact with each other and the resulting Teflon sheet was designated as Teflon (1). On the other Teflon sheet, 0.164 g of the water absorbing resin particle agglomerates (1) having an average particle size of from 850 to 1200 $\mu$m were placed so as not to contact with each other and the resulting Teflon sheet was designated as Teflon (2).

The base material (Bemliese) was placed still on Teflon (1) and 3 ml of water was sprayed using an atomizer. Teflon (1) was then placed still upside-down on Teflon (2) so as to overlap the surface of the base material with the particle surface of Teflon (2). This was pressed down lightly by hand, left for 1 minute, and heated for 10 minutes at 180°C in an inert oven to yield an absorbent in which the water absorbing resin particle agglomerates (1) adhered to both sides of the base material.

The weight of the absorbent measured immediately after heating was 0.4061 g. The weight ratio of the water-absorbing resin in the absorbent was calculated as 80.4%. All of the water-absorbent resin particle agglomerates (1) strongly adhered to the base material (Bemliese) and none of the agglomerates became detached when rubbed by hand. The adhesion state was observed with a scanning electron microscope ("JSM-5300", product of JEOL), and it was found that all the particles adhered to the base material, with fibers incorporated inside the water absorbing resin. The absorbent had an absorption ratio of 54.1 (g/g) and an absorption ratio after one minute of 7 (g/g).

[Table 4]

| | Density (g/m$^2$) | Absorption ratio (g/g) | Contact angle (degree) | Absorption speed (mg/s) | Thickness (mm) | Tensile breaking strength (N/20 mm) | Elongation (cm) | Tensile breaking strength after absorption of physiological saline (N/cm$^2$) |
|---|---|---|---|---|---|---|---|---|
| Bemliese lengthwise | 28 | 14 | 0 | 0.74 | 0.45 | 7.2 | 4 | 4.9 |
| Bemliese widthwise | | | | 0.58 | | 1.5 | 12.3 | 1 |

Industrial Applicability

The manufacturing method of water absorbing resin particle agglomerates and the water absorbing resin particle agglomerates according to the present invention can be used widely in the manufacturing fields of absorbents to be used in the fields of hygiene materials, agriculture and forestry, and civil engineering.

They are particularly suited for use in the production fields of absorbents of paper diapers, sanitary napkins and the like.

The following numbered paragraphs represent embodiments of the invention:

1. A manufacturing method of water absorbing resin particle agglomerates, which comprises the following steps (1) and (2):

(1) a polymerization step for producing primary particles of a water absorbing resin comprising suspending an aqueous monomer solution containing an unsaturated carboxylate in an organic solvent containing a nonionic surfactant therein, and subjecting the resulting suspension to reverse-phase suspension polymerization; and
(2) an agglomeration step of agglomerating the primary particles by using a water soluble solvent.

2. The manufacturing method of water absorbing resin particle agglomerates according to Claim 1, wherein the nonionic surfactant has an HLB of from 4 to 12.

3. The manufacturing method of water absorbing resin particle agglomerates according to Claim 1 or 2, wherein the water soluble solvent is a monoalcohol and/or a polyvalent alcohol having two or more alcohol groups.

4. The manufacturing method of water absorbing resin particle agglomerates according to any one of Claims 1 to 3, wherein the monomer concentration of the aqueous monomer solution at the time of initiation of the polymerization in Step (1) is from 40 to 80 wt.%.

5. The manufacturing method of water absorbing resin particle agglomerates according to any one of Claims 1 to 4, wherein ammonium salts constitute from 60 to 100 mol% of total amount of unsaturated carboxylic acids and salts thereof in the aqueous monomer solution in Step (1).

6. The manufacturing method of water absorbing resin particle agglomerates according to any one of Claims 1 to 5, wherein the unsaturated carboxylate in the aqueous monomer solution in Step (1) is ammonium (meth)acrylate.

7. The manufacturing method of water absorbing resin particle agglomerates according to any one of Claims 1 to 6, further comprising a fusion bonding step of keeping the suspension at a temperature of 40°C or greater after formation of the agglomerates.

8. The manufacturing method of water absorbing resin particle agglomerates according to any one of Claims 1 to 7, further comprising a drying step of drying the water absorbing resin agglomerates and a heating step of heating the resulting water absorbing resin agglomerates.

9. The manufacturing method of water absorbing resin particle agglomerates according to Claim 8, wherein the heating temperature in the heating step is from 130 to 250°C.

10. Water absorbing resin particle agglomerates of comprising primary particles consisting of water absorbing resin and satisfying the following requirements (a) and (b):

(a) 50 mol% or greater of repeating units of the polymer molecular chain of the water absorbing resin constituting the primary particles are carboxyl group-containing units and at least a portion of carboxyl groups of the carboxyl group-containing units is neutralized with at least one base selected from alkali metals, amines, and ammonia, and
(b) the water absorbing resin particle agglomerates comprise, on the outer surface thereof, a portion having a neutralization ratio of carboxyl groups of not greater than 40 mol% and, inside of the water absorbing resin particle agglomerates, a portion having a neutralization ratio of carboxyl groups of 50 mol% or greater.

11. The water absorbing resin particle agglomerates according to Claim 10, that have an average particle size of from 100 to 5000 $\mu$m.

12. The water absorbing resin particle agglomerates according to Claim 10 or 11, wherein the primary particles have an average particle size of from 30 to 1000 $\mu$m.

13. The water absorbing resin particle agglomerates according to any one of Claims 10 to 12, wherein 50 mol% or greater of the carboxyl-neutralized salt in the polymer molecular chain of the water absorbing resin constituting the primary particles are ammonium salts.

14. Body-fluid absorbing articles comprising the water absorbing resin particle agglomerates produced by the manufacturing method as claimed in any one of Claims 1 to 9 or the water absorbing resin particle agglomerates as claimed in any one of Claims 10 to 13.

**Claims**

1.  Water absorbing resin particle agglomerates of comprising primary particles consisting of water absorbing resin and satisfying the following requirements (a) and (b):

    (a) 50 mol% or greater of repeating units of the polymer molecular chain of the water absorbing resin constituting the primary particles are carboxyl group-containing units and at least a portion of carboxyl groups of the carboxyl group-containing units is neutralized with at least one base selected from alkali metals, amines, and ammonia, and
    (b) the water absorbing resin particle agglomerates comprise, on the outer surface thereof, a portion having a neutralization ratio of carboxyl groups of not greater than 40 mol% and, inside of the water absorbing resin particle agglomerates, a portion having a neutralization ratio of carboxyl groups of 50 mol% or greater.

2.  The water absorbing resin particle agglomerates according to Claim 1, that have an average particle size of from 100 to 5000 $\mu$m.

3.  The water absorbing resin particle agglomerates according to Claims 1 or 2, wherein the primary particles have an average particle size of from 30 to 1000 $\mu$m.

4.  The water absorbing resin particle agglomerates according to any one of Claims 1 to 3, wherein 50 mol% or greater of the carboxyl-neutralized salt in the polymer molecular chain of the water absorbing resin constituting the primary particles are ammonium salts.

5.  Body-fluid absorbing articles comprising the water absorbing resin particle agglomerates as claimed in any one of Claims 1 to 4.

Europäisches
Patentamt

European
Patent Office

Office européen
des brevets

**EUROPEAN SEARCH REPORT**

Application Number

EP 10 15 5064

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 02 194010 A (IDEMITSU PETROCHEMICAL CO) 31 July 1990 (1990-07-31) * abstract * | 1-5 | INV. C08F20/06 A61L15/22 C08J3/16 |
| X | JP 07 070245 A (NIPPON SYNTHETIC CHEM IND) 14 March 1995 (1995-03-14) * abstract * | 1-5 | |
| X | JP 2005 200630 A (ASAHI CHEMICAL CORP) 28 July 2005 (2005-07-28) * abstract * | 1-5 | |
| A | JP 2001 002713 A (MITSUBISHI CHEM CORP) 9 January 2001 (2001-01-09) * abstract * | 1-5 | |
| X | EP 1 512 417 A (NIPPON CATALYTIC CHEM IND [JP]) 9 March 2005 (2005-03-09) * paragraph [0105] - paragraph [0108]; claims 5,9,13 * * paragraphs [0001], [0018], [0205], [0206] * | 1-5 | TECHNICAL FIELDS SEARCHED (IPC) C08F A61L C08J |
| X | WO 99/25393 A2 (AMCOL INTERNATIONAL CORP [US]) 27 May 1999 (1999-05-27) * page 92; example 3; table 11 * | 1-5 | |

The present search report has been drawn up for all claims

| Place of search | Date of completion of the search | Examiner |
|---|---|---|
| Munich | 12 May 2010 | Gold, Josef |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

**ANNEX TO THE EUROPEAN SEARCH REPORT
ON EUROPEAN PATENT APPLICATION NO.**                    EP 10 15 5064

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2010

| Patent document cited in search report | | Publication date | Patent family member(s) | | Publication date |
|---|---|---|---|---|---|
| JP 2194010 | A | 31-07-1990 | NONE | | |
| JP 7070245 | A | 14-03-1995 | NONE | | |
| JP 2005200630 | A | 28-07-2005 | NONE | | |
| JP 2001002713 | A | 09-01-2001 | NONE | | |
| EP 1512417 | A | 09-03-2005 | CN | 1597787 A | 23-03-2005 |
| | | | US | 2005049379 A1 | 03-03-2005 |
| WO 9925393 | A2 | 27-05-1999 | AU | 1522199 A | 07-06-1999 |
| | | | BR | 9814686 A | 20-11-2001 |
| | | | CA | 2310691 A1 | 27-05-1999 |
| | | | CN | 1286635 A | 07-03-2001 |
| | | | EP | 1042013 A2 | 11-10-2000 |
| | | | FI | 20001087 A | 28-06-2000 |
| | | | HR | 20000308 A2 | 31-12-2000 |
| | | | HU | 0101141 A2 | 28-08-2001 |
| | | | ID | 30075 A | 01-11-2001 |
| | | | JP | 2001523733 T | 27-11-2001 |
| | | | NO | 20002546 A | 20-06-2000 |
| | | | PL | 340959 A1 | 12-03-2001 |
| | | | SI | 20362 A | 30-04-2001 |
| | | | SK | 7372000 A3 | 09-10-2000 |
| | | | TR | 200001461 T2 | 21-08-2001 |
| | | | US | 6555502 B1 | 29-04-2003 |
| | | | US | 6392116 B1 | 21-05-2002 |
| | | | US | 2001001312 A1 | 17-05-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO5584304 B
- JP SHO4943395 B
- JP SHO51125468 B
- JP SHO5214689 B
- JP SHO5315959 B
- JP 2005200630 A
- JP HEI66612 B
- JP HEI117482 B

- JP SHO6336322 B
- JP SHO6336321 B
- JP SHO62132936 B
- JP HEI326204 B
- US 6586534 B
- US 6174946 B
- JP HEI977810 B
- JP 2004305062 A